# EUROPEAN PATENT APPLICATION

(11) **EP 4 685 747 A1**
(43) Date of publication of application: **28.01.2026**
(21) Application number: 25185867.6
(22) Date of filing: 27.06.2025
(51) Int. Cl.: G06T 7/73

(54) **METHOD AND SYSTEM FOR GENERATING A MODEL FOR USE IN INTRAORAL NAVIGATION IN A PATIENT**

(30) Priority: 26.07.2024 DE 102024121325
(71) Applicant: Institut Straumann AG, 4002 Basel (CH)
(72) Inventor: Schnappauf, Albrecht, Basel (CH)
(74) Representative: Fleuchaus & Gallo Partnerschaft mbB

(57) **Abstract**

A computer implemented method (100) for generating, by one or more computer processors, a 3-dimensional model (10D) for use in assisted navigation of the oral cavity of a patient, the method based on at least a first scan of an anatomical region of the patient's oral cavity, the method comprising: receiving a first dataset including surface boundary information from the first scan and generating a 3-dimensional digital patient model from at least a portion of the first dataset, performing a 3-dimensional surface scan of a part of the intraoral cavity using a 3-dimensional surface scanner and thereby acquiring a 3-dimensional surface scan, the 3-dimensional surface scan including a region of interest, matching the acquired 3-dimensional surface scan to at least a portion of the 3-dimensional digital patient model, determining, employing the acquired 3-dimensional surface scan and the digital patient model, a spatial transformation configured to align or bring into a predetermined degree of registration the acquired 3-dimensional surface scan and the 3-dimensional digital patient model, determining a position and orientation information of the 3-dimensional surface scanner relative to the 3-dimensional digital patient model using at least the transformation, and generating a second dataset including the position and orientation information of the 3-dimensional surface scanner relative to the 3-dimensional digital patient model.

## Description

### Field of the Invention

The present invention relates to a method and system for generation of a model for use in oral cavity navigation in a patient, of particular but by no means exclusive application in planning a dental procedure.

### Background of the Invention

Digital software tools for dental procedures and planning employ accurate scan data of the oral cavity, which can include anatomical objects of interest (e.g. teeth) and anatomical structures such as bone and gum tissue, but also artificial structures such as dental protheses, abutments and anchoring systems. Accurate scan data of the oral cavity facilitates construction and positioning of, in particular, dental protheses and anchoring systems.

In the field of digital dental technology, scan data of anatomical structures are generally determined optically or radiologically. Optical scanners (i.e. those employing visible wavelengths and in some cases infrared and/or UV wavelengths) for three-dimensional measurement directly from intra-oral surface structures, or from extra-oral impressions of oral surface structures, are widespread and economical. Usually, surface data is represented by a surface mesh comprising triangular elements, which are routinely saved and exchanged between systems in STL or similar digital surface definition formats.

Radiological scanners such as digital volume tomographs (DVT) or computer tomographs (CT) use X-rays to generate volume datasets of anatomical structures. Surface representations can also be determined from these datasets by applying thresholding methods in which the intensity values (measured, for example, in Hounsfield units) of the individual scan elements (voxels) are analyzed to determine whether they exceed or fall below certain thresholds. Radiologically dense structures (hereinafter "volumetric density" structures or objects) such as teeth can be identified in this way and the boundary surface of the identified volumetric density scan structures can be modeled as triangulated surface data (e.g. a triangular surface mesh) and again be saved and exchanged between systems in STL or similar digital surface definition formats.

Computations with MRI (Magnetic Resonance Imaging) scan data are more complex, in which contour analysis methods are preferably applied, which work on the basis of the gradients of adjacent scan elements. However, also in these cases, volumetric structures or objects such as gum, gingiva, and other tissue, predominantly soft tissue, can be identified and its boundary surface modeled as triangulated surface data for further processing.

For the above scanning methods, such as, surface scanning using infrared, visible, or UV radiation, as well as volumetric methods, such as, radiological methods, for example, DVT or CT, and, similarly, MRI, there exist so called marker elements that are designed to be clearly visible-sometimes even having features helping to determine not only their position and orientation with higher precision-in the three-dimensional measurement data of one or multiple of the respective surface or volumetric scanning methods. Thus, they can be used to define exact landmarks within the data set, when placed and fixed in position relative to the dentition or jaw bone of a patient before scans are taken. This way, marker elements can enable the definition of a reference frame within the respective scan data with high precision.

In the context of the present disclosure, the term tooth restoration includes every type of tooth replacement, in part or in full, of single or multiple teeth. The term virtual tooth restoration (referred to more briefly as tooth restoration in the following) is to be understood to include appropriate electronic representations of tooth restorations, i.e. digital three-dimensional representations, preferably surface representations, of the jawbone, gum tissue, marker elements, anchoring systems, anchoring systems, and the likes of sufficient accuracy.

When designing customer prosthetics, prosthetics are often designed to replace an anatomical object that was removed from the existing oral situation. In addition to the importance of the precision in the manufacture of dental prosthetics (e.g. crowns), preparative steps (e.g. drilling of a socket for anchoring systems) must be performed with very high precision and it is necessary to model the patient's anatomical situation exactly for appropriate planning of the prosthetics. Likewise, it is necessary to reproduce the positioning of any elements, such as anchoring systems, placed in the model of the patient's anatomical situation at the planning step in the corresponding position of the patient's actual anatomy with very high accuracy, both in terms of position and orientation.

To help achieving the latter, existing techniques employ placement and affixation of marker elements in dental templates, and or relative to the patient's dentition or jaw bone, allowing to span a reference frame in the digital model of the patient's dental status. This reference frame provides the basis for digital implant planning and, thereafter, for the manufacturing of a drill guide, such as a 3D-printed, moulded, or thermoformed template which can placed over the patient's remaining teeth, screwed to the jawbone, or otherwise be anchored in place, and including one or multiple guide aperture for controlling the correct orientation and location of a drill and subsequently a dental pin for anchoring a dental prosthetic. This process is very complex, and thus expensive, usually requires multiple visits by the patient to the doctor's office and, despite the achievements in precision, still includes numerous sources of error.

There is an increasing demand for more immediacy and lower cost in dental treatments. In particular, for procedures that can be completed in one or at least fewer office visits than would have been possible in the past, novel ways to shorten dental treatment planning and execution are being sought. It is therefore an object of the present invention to alleviate at least some of the disadvantages of known dental restoration procedures.

### Summary

The object is solved at least in part by a computer implemented method for generating a 3-dimensional model for use in navigation of the oral cavity of a patient. An embodiment comprises a method for generating such a 3-dimensional model according to independent claim 1. Another embodiment provides a system for a generating 3-dimensional model according to independent claim 16.

Dependent claims 2 to 15 represent various embodiments of the invention.

According to an aspect of the invention, there is provided a computer implemented method for generating, by one or more computer processors, a 3-dimensional model for use in assisted navigation of the oral cavity of a patient, the method based on at least a first scan of an anatomical region of the patient's oral cavity. The method comprises:
receiving a first dataset including surface boundary information from the first scan (which, it should be noted, may include surface scan data obtained from a surface scan or generated from volumetric density scan data);
generating a 3-dimensional digital patient model from at least a portion of the first dataset;
performing a 3-dimensional surface scan of a part of the intraoral cavity with a 3-dimensional surface scanner and thereby acquiring a 3-dimensional surface scan, the 3-dimensional surface scan including a region of interest;
matching the acquired 3-dimensional surface scan to at least a portion of the 3-dimensional digital patient model;
determining, employing the acquired 3-dimensional surface scan and the digital patient model, a spatial transformation configured to align or bring into a predetermined degree of registration the acquired 3-dimensional surface scan and the 3-dimensional digital patient model;
determining a position and orientation information of the 3-dimensional surface scanner relative to the 3-dimensional digital patient model using at least the transformation; and
generating a second dataset including the position and orientation information of the 3-dimensional surface scanner relative to the 3-dimensional digital patient model.

The anatomical region typically includes at least one type of tissue of the patient. The types of tissue in the anatomical region of the patient may comprise bone and/or dental tissue (i.e. enamel, dentin and/or cementum), but also soft tissue (e.g. gum tissue). Likewise, the 3-dimensional digital patient model may include one or more portions of jawbone, of gum tissue and of one or more teeth.

The invention recognizes that the dental surfaces of a patient represent a very unique and highly detailed topology which can be used to accurately place an object relative thereto with very high precision. Accordingly, once a detailed 3-dimensional digital model of the patient's anatomy has been created, a navigation assist system can inform a user or a robotic dental treatment system very precisely of the position and orientation of a 3-dimensional surface scanner's field of view (and thus the device itself) relative to the patient's dentition (or an imprint thereof) based on the surface scan acquired by said 3-dimensional surface scanner of a patient's anatomy or, respectively, its dental imprint.

Moreover, given the first dataset may comprise surface boundary information of the patient's jawbone, and thus the digital patient model also represent the jawbone relative to the patient's dentition in an accurate manner, the method of the invention also serves to inform the position and orientation of a 3-dimensional surface scanner's field of view relative to such jaw bone of patient, even if there is no visibility of the bone structure in the 3-dimensional surface scanner's field of view.

Accordingly, using the invention, the use of marker elements can be avoided or at least substantially reduced and need no longer be used to help in the process of digital treatment planning of a dental procedure or to create drill guides for placement of dental anchoring systems but a more immediate feedback and assisting in the treatment can be provided by allowing a user, or alternatively a robotic navigation and treatment system, to determine its exact position relative to a patient's dentition or oral anatomy. Indeed, even the exposure to x-rays can, under certain circumstances, be minimized by employing the system of the invention.

In an embodiment, the second dataset further includes at least a portion of the 3-dimensional digital patient model.

In an embodiment, the surface boundary information of the first dataset includes surface segments, the surface segments having labels associated therewith identifying the type of the tissue, each surface segment represents, the type of tissue being selected from a group comprising gum tissue, bone tissue, and dental tissue.

This may comprise identifying elements of the anatomical region of the patient's oral cavity in the 3-dimensional digital patient model, i.e. by a user or a suitably trained artificial neural network, in the context of executing the method of the invention but may also comprising retaining labels associated with surface segments identifying the type of tissue in the process of generating the 3-dimensional digital patient model, that is, retaining previously assigned labels associated with such surface segment, for example, when the first dataset is based on a previously obtained volumetric density scan and post edited.

The surface boundary information of the first dataset be have been generated from a set of density scan segments from the second dataset that is cross-mounted in a common 3D coordinate system with a set of corresponding surface scan segments from a surface scan, where the cross-mounting may be implemented by using correspondingly labeled surface scan segments and matching these, preferable geometrically, to volumetric density scan segments, such as, for example, by determining surface scan segments and volumetric density scan segments of similar shape and volume. Thus where, in the prior art, such cross-mounting may have relied on the position and orientation information of one or multiple marker elements within the three-dimensional measurement data, the use of such marker elements may be avoided in the application of the present invention. The present invention may include scaling, translatory and rotatory transformations applied to the surface scan segments and/or volumetric density scan segments to achieve the best possible registration of the surface scan segments and volumetric density scan segments in the common 3D coordinate system where, in particular, elements from both the surface and the volumetric scans indicating the dental surface of the person's dentition may be employed in the process of cross-mounting the surface scan segments with the volumetric density scan segments, that is, putting the segments of both scans in close registration where, for example, a geometric overlap exists.

The second dataset may comprise a digital representation of the 3-dimensional surface scanner in a common reference frame, the digital representation of the 3-dimensional surface scanner being positioned and oriented according to the position and orientation. The digital representation of the 3-dimensional surface scanner may be schematic, i.e. by including a 3-dimensional surface model of the geometry of the 3-dimensional surface scanner, the 3-dimensional surface model the surface scanner being positioned and oriented corresponding to the 3-dimensional surface scanner's position and orientation at the time the 3-dimensional surface scan of the region of interest was taken.

In an embodiment, the second dataset may be transferred to a visualization system configured to visualize at least a portion of the second dataset to a user in a 2-dminesional, a pseudo-3-dimensional, or a 3-dimensional image representation. The visualization system may be further configured to augment or overlay the display of the portion of the second dataset with a view of the patient's oral cavity.

These embodiments enable a user to get a very fluid and natural view of the patient's oral cavity (either through an image representation or a live view) and, at the same time, give the user visual feedback of the position and orientation of the 3-dimensional surface scanner inside the patient's oral cavity and relative to the patient's tech and jaw.

In an embodiment, the second dataset may be generated as a 3-dimensional output model, allowing simplified post processing of the information contained in the second dataset, for example, in the process of designing dental protheses or when generating a treatment or inspection plan for a dental procedure that includes placement of a dental anchoring system.

In an embodiment, a region of interest may be determined in the 3-dimensional output model based on the dental treatment or inspection plan, and the region of interest may be identified in the 3-dimensional output model, such as by application of a specific label to portions of the 3-dimensional output model that lie within the region of interested and thus allowing, for example, an easier highlighting of such portions in the output of the visualization system, or a definition of a "no-go" zone where the 3-dimensional output model is loaded into a robotic navigation and treatments system for conducting a dental procedure through a robotic (assist) system.

In one example, the method comprises generating the second dataset as a 3-dimensional output model. For example, the method may comprise transferring the second dataset to a navigation system configured to display the 3-dimensional output model. The navigation system may, in addition to the 3-dimensional patient model, receive a dental treatment or inspection plan, determine one or more deviations of the 3-dimensional output model from the dental treatment or inspection plan, and identify the deviations in the 3-dimensional output model.

In an embodiment, the method comprises acquiring and updating the 3-dimensional surface scan continuously or in real time. This warrants for an instant or near-instant update of the generation of the second dataset and thus helps to make the experience of using, for example, a (robotic) visualization and navigation system in conjunction with such embodiment a near-real one for the user. As such, the hurdles of using such a system can be reduced by which the benefit becomes particularly pronounced to the patients due to the increased navigational precision that can be achieved with the system of the invention.

In an embodiment, the 3-dimensional surface scanner may comprise, be integral with or be mounted on a dental instrument, such as, a dental drill, a dental probe, or any other dental instrument suitable for determining the dental status of a patient or manipulating a patient's teeth, gum or jawbone. Such embodiment is particularly useful if the invention is being used as a navigation assist system for a user during dental inspection or treatment procedures as it's warranted that the field of view of the 3-dimensional surface scanner always exactly "sees" the portion the dental instrument would be engaging with.

In a further embodiment, as has been indicated above, the invention comprises transferring the second dataset to a robotic navigation system configured to control, or assist in controlling, navigation of a dental instrument, such as a dental drill, a dental probe, or another type of dental instrument, based on the position and orientation information included in the second dataset.

In an embodiment, the matching of at least a portion of the 3-dimensional digital patient model to the acquired 3-dimensional surface scan employs only dental surfaces of the 3-dimensional digital patient model. Such matching could, for example, be conducted by applying a least square method to determine a best fit of the surface structures (topology) encoded in the 3-dimensional digital patient model to the 3-dimensional surface scan. Alternative known methods for determining best fits of topologies are known in the art and will thus not be elaborated further.

In a further embodiment, the matching of at least a portion of the 3-dimensional digital patient model to the acquired 3-dimensional surface scan employs only surface boundary information of the first dataset including segments having been labeled as dental tissue. This may enhance the determination of the best fit between the 3-dimensional digital patient model and the acquired 3-dimensional surface scan as the dental surfaces of a patient represent a unique and highly detailed and static topology which can be used as a basis for the high precision matching of the acquired 3-dimensional surface scan to the 3-dimensional digital patient model.

In an embodiment, the first dataset comprises labeled surface scan segments, each surface scan segment comprising a 3-dimensional surface model of a corresponding object recognized and segmented from the surface scan data, and each surface scan segment having an associated label identifying the surface scan segment. In such embodiments, the method comprises:
receiving a third dataset of labeled volumetric density scan segments, each volumetric density scan segment comprising a 3-dimensional volumetric density model of a boundary surface of a corresponding object recognized and segmented from volumetric density scan data of a volumetric density scan of the anatomical region, and each volumetric density scan segment having an associated label identifying the volumetric density scan segment;
cross-mounting labeled surface scan segments from the first dataset with labeled volumetric density scan segments from the third dataset in a common 3D coordinate system; and
generating the 3-dimensional digital patient model from at least the portion of the first dataset cross-mounted with the labeled volumetric density scan segments.

According to this aspect, there is also provided a computer implemented method for planning a dental procedure, comprising:
the method for generating, by one or more computer processors, the 3-dimensional model for use in navigation of the oral cavity of a patient, as described above; and
generating a superimposition dataset comprising a superimposition of at least a portion of the digital patient model and at least a portion of the 3-dimensional surface scan.

In an embodiment, the dental procedure comprises dental prothesis or dental prothesis support structure implantation, for example, dental anchoring systems.

According to another aspect, the invention provides a system for generating a 3-dimensional model for use in assisted navigation of the oral cavity of a patient. The system comprises: at least one computer processor; a storage unit; a 3-dimensional surface scanner for performing a 3-dimensional surface scan of a part of an intraoral cavity of a patient so as to acquire a 3-dimensional surface scan that includes a region of interest; and, optionally, at least one visualization device and/or a robotic navigation (assist) system, wherein, the storage unit stores instructions which, when executed by the computer processor, implement the method of the invention of any of the aspects and/or embodiments as described above.

In particular, such system comprises a 3-dimensional digital patient model generator configured to generate a 3-dimensional digital patient model from at least a portion of a first dataset of surface boundary model data from an intraoral scan of an anatomical region of the patient's oral cavity;
a surface scan matcher configured to match the acquired 3-dimensional surface scan to at least a portion of the 3-dimensional digital patient model;
a spatial transformation determining engine configured to determine, employing the acquired 3-dimensional surface scan and the digital patient model, a spatial transformation configured to align or bring into registration the acquired 3-dimensional surface scan and the 3-dimensional digital patient model;
a scanner position and orientation determining engine configured to determine a position and orientation of the 3-dimensional surface scanner relative to the 3-dimensional digital patient model using at least the transformation; and
an output generator configured to output a second dataset including at least a portion of the 3-dimensional digital patient model and the position and orientation information of the 3-dimensional surface scanner relative to the 3-dimensional digital patient model.

In an embodiment, the system the output generator is configured to generate the second dataset in such way that the portion of the 3-dimensional digital patient model and the digital representation of the 3-dimensional surface scanner is transformed into in a common reference frame, in which the digital representation of the 3-dimensional surface scanner may be positioned and oriented according to the position and orientation determined by the scanner position and orientation determining engine.

In an embodiment, the system is configured to read continuously or in real time further 3-dimensional surface scan information from the 3-dimensional surface scanner and to update the 3-dimensional surface scan an thus the second dataset continuously or in real time.

In an embodiment, the 3-dimensional surface scanner comprises, is integral with or is mounted on a dental instrument, such as, a dental drill, a dental probe, or any other dental instrument suitable for determining the dental status of a patient or manipulating a patient's teeth, gum or jawbone.

The present invention takes benefit of the realization that the geometry and special position of the surface of a person's teeth is highly unique and may thus be regarded as a 3-dimensional topology of high precision which, in itself can be considered as defining a special reference frame. Thus, the use of marker elements in the process of obtaining any of the three-dimensional measurement data and generating the datasets based thereupon can be avoided. Accordingly, in an embodiment, the digital patient model of the present invention does not include a digital representation of a marker element.

In yet another aspect, the invention comprises computer program code which, when loaded into a storage device and executed by a computer processor in communicative connection with a 3-dimensional surface scanner for performing a 3-dimensional surface scan of a part of an intraoral cavity of a patient implements the method of the invention of any of the aspects and/or embodiments as described above in a suitably configured computer to create a system for generating a 3-dimensional model for use in assisted navigation of the oral cavity of a patient.

### Brief Description of the Drawing

The invention is further described by way of the following detailed description in conjunction with the drawing. In the following detailed description, like reference numerals denote like elements except where indicated otherwise.

While the following detailed description describes certain embodiments of the invention in greater detail, it is noted that features described in context with only one of the embodiments are nevertheless intended to be available also in the context of or in combination with any other embodiment of the invention, whether described or not in the detailed description, except where such a combination of features would lead to non-meaningful results.

In no way is the following detailed description meant to limit the invention to the specific embodiments and combinations of features therein; rather, the invention is exclusively limited and defined by the attached claims.
Figs. 1A is an architectural diagram of the system for generating a model for use in intraoral navigation in a patient according to an embodiment of the present disclosure;
Fig. 1B is an architectural diagram of the processor and memory of the system of Fig. 1A, according to an embodiment of the present disclosure;
Figs. 2A & 2B illustrate exemplary flow diagrams of methods for generating a model for use in intraoral navigation in a patient according to embodiments of the present disclosure;
Figs. 3A to 3O illustrate an exemplary embodiment of a method for generating a model for use in intraoral navigation in a patient according to embodiments of the present disclosure;
Figs. 4A, 4B, 5A & 5B depict a first exemplary graphical user interface implementation of various aspects of the present disclosure;
Figs. 6A to 6J depict a second exemplary graphical user interface implementation of various aspects of the present disclosure;
Figs. 7A to 7T depict a third exemplary graphical user interface implementation of various aspects of the present disclosure.

### Detailed Description

An aspect of the invention is the generation of 3-dimensional model for use in navigation of the oral cavity of a patient. An embodiment comprises a method for generating such a 3-dimensional model.

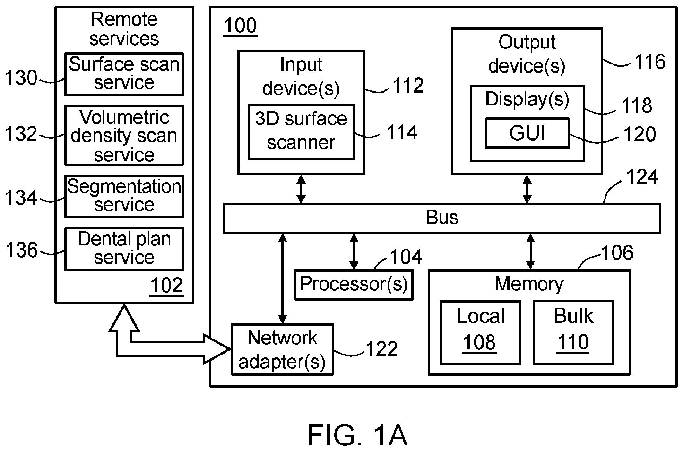

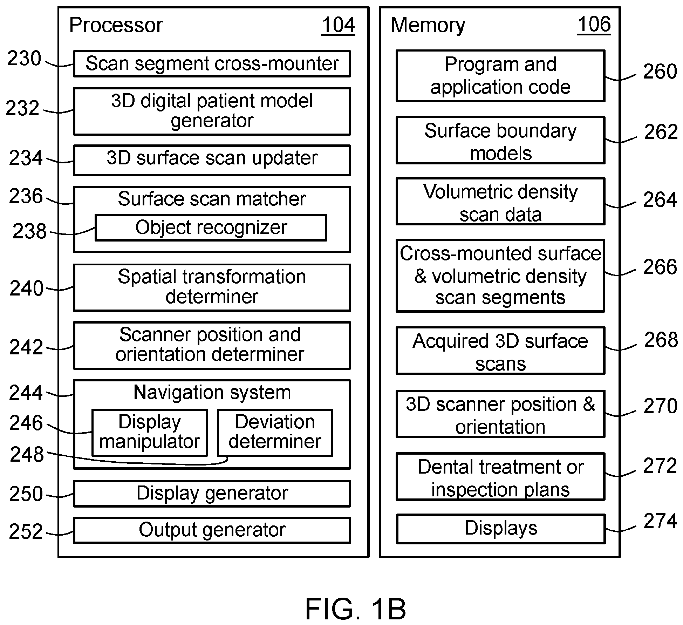

FIG. 1A is an architectural diagram of a computer-enabled system 100 for generating a model for use in intraoral navigation in a patient according to an embodiment of the present disclosure, shown with external or remote services 102. FIG. 1B is an architectural diagram of the processor 104 and memory 106 of system 100 of FIG. 1A, according to an embodiment of the present disclosure.

Referring to FIGS. 1A and 1B, system 100 includes at least one processor 104, memory 106 (which includes local memory 108 and bulk memory 110), one or more input devices 112 (including an intra-oral scanner in the form of a 3-dimensional surface scanner 114), one or more output devices 116 (including at least one display 118 on which is generated a graphical user interface (GUI) 120), and one or more network adapters 122. System 100 also includes a bus 124 for facilitating communication between processor 104, memory 106, input devices 112, output devices 116 and network adapters 122. It should be noted that 3-dimensional surface scanner 114 is in data communication with bus 124, and that this data communication may be wired or wireless; in the latter case, data communication is facilitated by a wireless protocol (not shown), such as Bluetooth (trade mark) or Wi-Fi (trade mark).

Remote services 102 include a surface scan service 130, a volumetric density scan service 132, a segmentation service 134, and dental plan service 136.

Processor 104 implements programs and applications read from memory 106 that implement various functions of system 100, as described below. Thus, processor 104 includes a scan segment cross-mounter 230, a 3-dimensional digital patient model generator 232, a 3-dimensional surface scan up-dater 234, a surface scan matcher 236 (which includes, for example, an object recognizer 238 that implements object recognition), a spatial transformation determiner 240, a scanner position and orientation determiner 242, a navigation system 244 (which includes a display manipulator 246 and a deviation determiner 248), a display generator 250 and an output generator 252.

Memory 106 includes program and application code 260 that is configured to be read and executed by processor 104, such that processor 104 implements the various functions described above. Memory 106 also includes surface boundary models 262 (which may comprise labeled surface scan segments) received from surface scan service 130 or volumetric density scan service 132 of remote services 102, volumetric density scan data 264 (which may comprise labeled volumetric density scan segments) received from volumetric density scan service 132 of remote services 102, cross-mounted surface and volumetric density scan segments 266 (generated and outputted by scan segment cross-mounter 230), acquired 3-dimensional surface scans 268 (received from 3-dimensional surface scanner 114), 3-dimensional scanner position & orientation 270 (generated and outputted by scanner position and orientation determiner 242), dental treatment or inspection plans 272 (received from external dental plan service 136) and displays 274 (generated and outputted by display generator 250).

Network adapters 122 facilitate communication between system 100 and remote services 102.

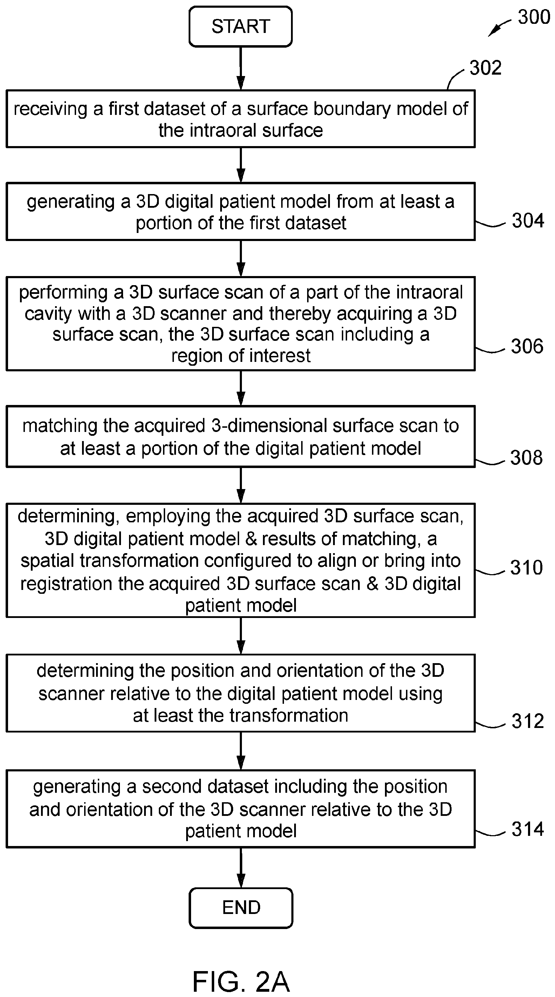

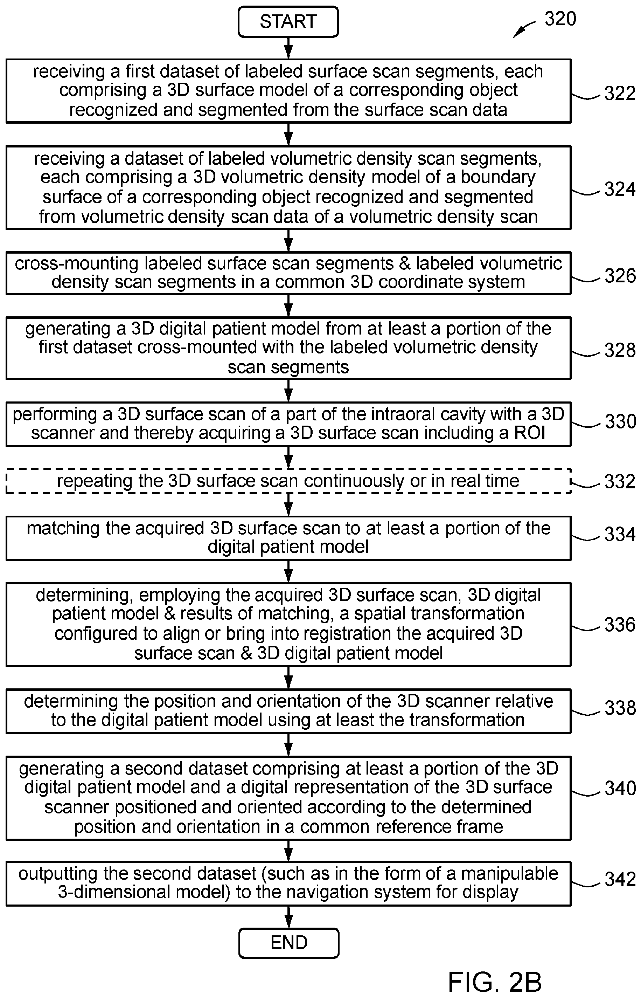

FIGS. 2A and 2B illustrate exemplary flow diagrams of methods 300 and 320, respectively, for generating a model for use in intraoral navigation in a patient according to embodiments of the present disclosure. Method 300 of FIG. 2A may be regarded as a simple method for generating such a model, while method 320 of FIG. 2B may be regarded as a complex method for generating such a model, but it should be appreciated that methods 300 and 320 have several common steps and embody the same core techniques.

Referring to FIG. 2A, method 300 is a method for automatically generating, by processor 104, a 3-dimensional model for use in navigation of the oral cavity of a patient. To do so, method 300 employs on at least an intraoral surface scan of an anatomical region of the patient's oral cavity. The anatomical region includes at least one tissue of the patient.

At step 302, system 100 receives a first dataset of surface boundary model data from an intraoral scan. The first dataset may be surface scan data may be obtained from a surface scan and received from remote surface scan service 130 of remote services 102. Alternatively, the first dataset may be generated from volumetric density scan data by and received from volumetric density scan service 132 of remote services 102.

Step 302 may include storing the first dataset in surface boundary models 262.

The first dataset, in this embodiment, comprises one or more labeled surface scan segments, each comprising an individual segment corresponding to objects and/or features recognized, via image recognition and segmentation processing (such as by segmentation service 134), in a surface scan of the oral cavity and, in particular, the anatomical region of interest. In this embodiment, the surface scan data is generated by remote surface scan service 130, which may do any one or any combination of the following: collection of surface scan data collected from a surface scanner (such as an optical scanner or camera), converting the surface scan data into a 3-dimensional model which can be accessed and read by the processor(s) 104 of the system 100, manipulated or converted into another format, if necessary, to ready it for display on display 118, and saved in a computer-readable file as surface boundary models 262 of memory 106. In an alternative embodiment, system 100 includes surface scan service 130; in such embodiments, method 300 includes the steps performed by surface scan service 130.

Remote services 102 may also include segmentation service 134, configured to receive scan data (whether surface (IOS) or volumetric density (CBCT or CT) scan data) and use image processing, extraction and classification to segment received scan images containing an anatomical area of interest into various identified objects and associating a classification tag to each of the segment. Segmentation service 134 may provide the segments in the form of individual virtual 3D segment models, where each individual virtual 3D segment model (herein also referred to as simply "segment model") is a digital 3D representation of the actual anatomical part or feature of the patient's anatomy. In an embodiment, segmentation service 134 provides each segment as an individual digital 3D model, preferably (but not limited to) in STL format as a triangular mesh or a point cloud.

In an alternative embodiment, system 100 includes segmentation service 134. In such an embodiment, method 300 includes the steps performed by segmentation service 134.

At step 304, 3-dimensional patient model generator 232 generates a 3-dimensional digital patient model from at least a portion of the first dataset.

GUI 120 may include a 3D model view pane for displaying the 3-dimensional digital patient model (in this embodiment based on the surface scan of the area of interest). GUI 120 includes various user controls to allow the user to direct display generator 250 to perform various operations, such as but not limited to: selecting and loading a patient's scan records, selecting and manipulating display views, selecting content for display in GUI 120, selecting objects of interest and/or identifiers of objects of interest that may be included in a patient's scan data, selecting and viewing identifiers, descriptions and images of implants, prosthetics, materials, etc. in connection with planning dental treatment for a patient, and so on.

At step 306, a 3-dimensional surface scan is performed of at least a part of the intraoral cavity of the patent with 3-dimensional surface scanner 114, thereby acquiring a 3-dimensional surface scan that includes a region of interest. The region of interest may be or include, for example a diseased tooth or the proposed site of a dental implant or prosthesis.

At step 308, surface scan matcher 236 matches the 3-dimensional surface scan acquired in step 306 to at least a portion of the 3-dimensional digital patient model generated in step 304, such as by identifying features common to both using object or feature recognition (implemented by object recognizer 238). Surface scan matcher 236 outputs the results of this process, such as in the form of data associating pixels or voxels of the 3-dimensional surface scan acquired in step 306 found to match pixels or voxels of the 3-dimensional digital patient model.

At step 310, spatial transformation determiner 240 determines, employing as inputs the 3-dimensional surface scan acquired in step 306, the 3-dimensional digital patient model generated in step 304 and the output of surface scan matcher 236, a spatial transformation configured to align or bring into registration the acquired 3-dimensional surface scan and the 3-dimensional digital patient model.

At step 312, scanner position and orientation determiner 242 determines the position and orientation of 3-dimensional surface scanner 114 relative to the 3-dimensional digital patient model using at least the transformation. At step 314, generator 252 generates a second dataset including the position and orientation of the 3-dimensional surface scanner 114 relative to the 3-dimensional digital patient model. The second dataset may be output to a storage device, such as a memory or a non-transitory storage device. The storage device may be remote of system 100, or consist of a graphics memory of system 100 (or of a remote system) so that the position and orientation can be used in the generation of a display.

It will be noted that method 300 employs only surface scan data (acquired from surface scan service 130) pertaining to the patient when generating the 3-dimensional digital patient model. Method 320 of FIG. 2B is also a method for automatically generating, by processor 104, a 3-dimensional model for use in navigation of the oral cavity of a patient employing an intraoral surface scan of the anatomical region of the patient's oral cavity. Method 320, however, additionally employs volumetric density scan data (from, for example, CT or CBCT scans) from volumetric density scan service 132 when generating the 3-dimensional digital patient model.

Thus, referring to FIG. 2B, at step 322, system 100 receives a first dataset of surface scan data (in the form of labelled surface scan segments) of the intraoral surface from surface scan service 130 of remote services 102. Each segment comprises a 3-dimensional surface model of a corresponding object recognized and segmented from the surface scan data. This step may include storing the first dataset in surface boundary models 262. The first dataset is as described above in the context of method 300.

At step 324, system 100 receives a dataset of labeled volumetric density scan segments of the intraoral surface from volumetric density scan service 132 of remote services 102. Each volumetric density scan segment comprises a 3-dimensional volumetric density model of a boundary surface of a corresponding object recognized and segmented from volumetric density scan data of a volumetric density scan of the oral cavity of the patient. This step may include storing the dataset in volumetric density scan data 264.

The dataset of labeled volumetric density scan segments comprises individual segments corresponding to objects and/or features recognized, via image recognition and segmentation processing (such as by segmentation service 134), in one or more CT or CBCT volumetric density scans of the oral cavity and surrounding tissue, typically including the region of interest. The labeled volumetric density scan segments are generated by and received from remote volumetric density scan service 132. Volumetric density scan service 132 may do any one or any combination of the following: collection of volumetric density scan data collected from a volumetric density scanner (using volumetric scanning equipment such as cone-beam computer tomography (CBCT) scanner), converting the volumetric density scan data into a 3-dimensional model which can be accessed and read by processor 104 of system 100, manipulated or converted into another format, if necessary, to ready it for display on display 118, and saved in a computer-readable file that may be received by system 100 and stored in as volumetric density scan data 264 of memory 106.

In certain alternative embodiments, system 100 includes volumetric density scan service 132; in such embodiments, method 320 includes the steps performed by volumetric density scan service 132. Likewise, in embodiments in which system 100 includes segmentation service 134, method 320 includes the steps performed by segmentation service 134.

At step 326, scan segment cross-mounter 230 cross-mounts the labeled surface scan segments and the labeled volumetric density scan segments in a common 3-dimensional coordinate system. The received surface scan segments and volumetric density scan segments are generally obtained using different modalities (and therefore using different-and typically independent-scanning machines/equipment), so the scan data produced by each scan modality is collected and saved according to a 3D coordinate system native to the particular scanning machine/equipment that collected the data. It thus becomes important to align the resulting scan data from each scanner into a common 3-dimensional coordinate system such that like objects from each scan are matched and can be displayed as occupying the same spatial volume-as they should, since they each represent the same object or feature.

The result of the cross-mounting into a common 3-dimensional coordinate system is that, for each pair of surface scan and volumetric density scan segments that corresponds to the same patient object or feature from the scanned anatomical area of interest, there should exist one or a plurality of respective points that "match" (i.e., the points from each segment in the segment pair should substantially or exactly coincide) in the 3-dimensional space of the common 3-dimensional coordinate system. These matching points correspond to a corresponding point on the actual object/feature of the patient's actual anatomy. Matching points will only be present for areas of the patient's anatomy that the particular scan modality was able to capture.

Accordingly, since the surface scan data only includes surface visible image data and the volumetric scan data includes both surface and subsurface image data, point matching can only occur for points of the volumetric density scan segment(s) that correspond to surface-visible points of the scanned object (since the surface scan segment(s) contain no sub-surface data points). Ideally the points of each corresponding (or co-represented) pair of surface scan segment and associated volumetric density scan segment should exactly match in the common 3-dimensional coordinate system, but the points may only substantially match (that is, coincide within a margin of error), owing to differences in accuracy between the scan modalities as well as differences in resolution and generation accuracy of the 3-dimensional scan segment models generated for each scan modality. Nonetheless, cross-mounting step 326 should result in the surface scan segments corresponding to (or co-represented in) scanned objects occupying nearly identical space in the 3-dimensional coordinate system as like regions of the associated object as represented by corresponding volumetric density scan segments.

Thus, step 326 renders the two sets of segments (surface and volumetric) to be, for example, viewable in the common 3-dimensional coordinate system, overlaid or otherwise combined as desired.

At step 328, 3-dimensional patient model generator 232 generates a 3-dimensional digital patient model from at least a portion of the first dataset cross-mounted with the labeled volumetric density scan segments. At step 330, a 3-dimensional surface scan is performed of at least a part of the intraoral cavity of the patent with 3-dimensional surface scanner 114, thereby acquiring a 3-dimensional surface scan that includes a region of interest. The region of interest may be or include, for example a diseased tooth or the proposed site of a dental implant or prosthesis. Optionally, at step 332, the 3-dimensional surface scan is repeated or updated continuously or in real time with 3-dimensional surface scanner 114, such as to capture the surface scan evolving as 3-dimensional surface scanner 114 is moved in position or orientation.

The 3D model view pane of GUI 120 may be employed to display the 3-dimensional digital patient model (in this embodiment based on the surface scan and volumetric density scan data of the area of interest).

At step 334, surface scan matcher 236 matches the 3-dimensional surface scan acquired in step 330 (or as updated in step 332) to at least a portion of the 3-dimensional digital patient model generated in step 328, such as by identifying features common to both using object or feature recognition. Surface scan matcher 236 outputs the results of this process, such as in the form of data associating pixels or voxels of the 3-dimensional surface scan acquired in step 330 or 332 found to match pixels or voxels of the 3-dimensional digital patient model.

At step 336, spatial transformation determiner 240 determines, employing as inputs the 3-dimensional surface scan acquired in step 330/332, the 3-dimensional digital patient model generated in step 328 and the output of surface scan matcher 236, a spatial transformation configured to align or bring into registration the acquired 3-dimensional surface scan and the 3-dimensional digital patient model.

At step 338, scanner position and orientation determiner 242 determines the position and orientation of 3-dimensional surface scanner 114 relative to the 3-dimensional digital patient model using at least the transformation.

At step 340, generator 250 generates a second dataset comprising at least a portion of the digital patient model and a digital representation of the 3-dimensional surface scanner 114 in a common reference frame, with the digital representation of the 3-dimensional surface scanner being positioned and oriented according to the position and orientation determined in step 338.

The second dataset may be saved or outputted (such as by output generator 252) to displays 274 of memory 106, and is suitable for displaying on the screen of a computer or other computing device (such as with 3D model view pane of GUI 120 on display 118), so that a user may inspect the 3-dimensional digital patient model and the digital representation of the 3-dimensional surface scanner 114 correctly positioned and orientated relative to each other. Display generator 250 may optionally generate the second dataset as a manipulable 3-dimensional model.

The second dataset is especially useful in those embodiments in which 3-dimensional surface scanner 114 comprises, is integral with or is mounted on a dental drill, a dental probe, or other dental instrument, as it allows the user to determine whether 3-dimensional surface scanner 114 (and hence the dental instrument) is positioned and oriented as desired relative to the intraoral region of interest.

At step 342, display generator 250 outputs the second dataset (such as in the form of a manipulable 3-dimensional model) to navigation system 244. Navigation system 244 is configured to convert the second dataset into a display for displaying and manipulation with 3D model view pane of GUI 120. Display manipulator 246 of navigation system 244 provides GUI 120 with user controls to allow the user to control GUI 120 to perform various operations, such as but not limited to: selecting and loading a patient's scan records, selecting and manipulating display views (e.g. to rotate, expand or shrink the display), selecting content for display in GUI 120, selecting objects of interest and/or identifiers of objects of interest that may be included in a patient's scan data, selecting and viewing identifiers, descriptions and images of implants, prosthetics, materials, etc. in connection with planning dental treatment for a patient, and so on. Consequently, the user can navigate the 3-dimensional digital patient model, while viewing the model of 3-dimensional surface scanner 114 in the correct position and orientation relative to the 3-dimensional digital patient model including as the position and orientation evolve in response to user manipulation of the 3-dimensional surface scanner 114 and/or movements by the patient of his or her head or jaw.

Dental treatment or inspection plans are received from system 100 from remote dental plan service 136, and stored by system 100 in dental treatment or inspection plans 272 of memory 106. Deviation determiner 248 of navigation system 244 is configured to retrieve a dental treatment or inspection plan from dental treatment or inspection plans 272 or from dental plan service 136, to determine one or more deviations of the second dataset from the dental treatment or inspection plan, and to alter the display so as to identify those deviations. The deviations may be detected by surface scan matcher 236, using as inputs the respective dental treatment or inspection plan and the 3-dimensional digital patient model. Altering the display so as to identify the deviations may be effected by superimposing the deviations-such as by labeling or coloring regions of deviation-on the manipulable 3-dimensional model.

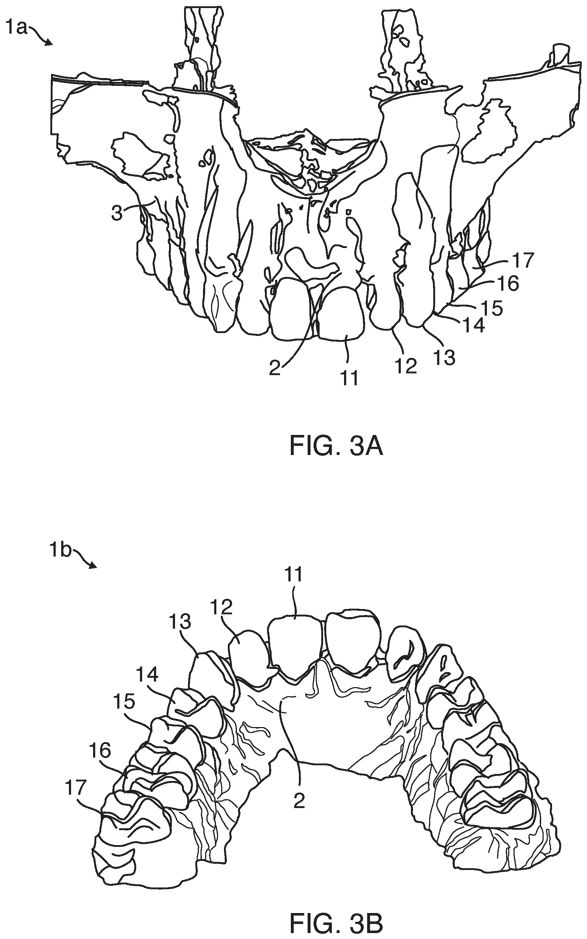

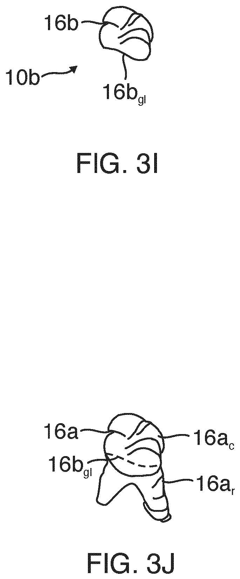

FIGS. 3A to 3O illustrate an exemplary embodiment of an aspect of the present invention according to which method 320 of FIG. 2B is employed in the generation of a 3-dimensional socket model from 3-dimensional surface scan segments (the 3D surface model of a corresponding object recognized and segmented from surface scan data of the surface scan) and volumetric density scan segments (the 3D volumetric density mode of a boundary surface of a corresponding object recognized and segmented from volumetric density scan data of the volumetric density scan) associated with an object. In the illustrated embodiment, the object is a tooth and the generated socket model is a tooth socket that is equivalent to the portion of the outer shape of the portion of the tooth that lies below the gumline, i.e. the tooth's root.

FIG. 3A shows an example of a 3D surface boundary model 1a generated based on a volumetric density scan. Individual structures in the model 1a correspond to actual structures of patient's oral situation. As shown, the 3D surface boundary model 1a embodies anatomical structure representations of a patient's actual gum tissue 2, bone 3, and teeth (shown labeled as 11, 12, 13, 14, 15, 16 and 17 according to Federation Dentair Internationale (FDI) notation (a commonly used teeth numbering system in the dental industry), among other individual teeth (not labeled). In an embodiment, surface boundary model 1a is generated from the 3D volumetric structures represented in the volumetric density scan and extracted through image recognition and segmentation techniques, such as, thresholding the intensity values (measured in Hounsfield units) of individual scan elements in the radiograph stack. In an embodiment, surface boundary model 1a comprises a point cloud, a triangular or other polygonal mesh, or other 3D digital model.

FIG. 3B shows an example of a 3D surface model 1b generated based on a surface scan of the same anatomical area as FIG. 3A. Individual structures in the model 1b correspond to actual structures of patient's oral situation. As shown, the 3D surface model 1b embodies anatomical structure representations of a patient's actual gum tissue 2, and teeth (shown labeled as 11, 12, 13, 14, 15, 16 and 17 according to FDI notation. Since the bone in the patient's oral cavity lies beneath the surfaces of the gum and teeth, typically no bone information would be present in the surface scan model 1b.

The surface scan and the volumetric density scan are obtained from scanning the same oral areas of interest, so both models 1a and 1b include respective model anatomical structures that represent certain same anatomical structure(s) (e.g., teeth 11, 12, 13, 14, 14, 15, 16 and 17, and gums 2) of the patient. Model 1b comprises a point cloud, a triangular or other polygonal mesh, or other 3D digital model, generated from the 3D surface structures represented in the surface scan.

As noted in FIGS. 3A and 3B, both the volumetric density scan model 1a and the surface scan model 1b are surface models of the (generally same) anatomical area. The 3D surface models 1a and 1b do not include representations of the internal anatomy. This means that neither model contains information about the sockets or other structure underneath the visible exterior surfaces of the objects in the model. In the illustrative example, where the object is a tooth, this means that one cannot ascertain the anatomy of the tooth socket which seats the tooth because the socket is not visible in either the 3D volumetric density scan model (FIG. 3A) or the 3D surface scan model (FIG. 3B).

In both models 1a in FIG. 3A and 1b in FIG. 3B, only the crown portions of the teeth are modeled; neither scan model includes the anatomy of the root or socket which seats the tooth. Although the surface detail is helpful when planning dental treatment for a patient, for planning surgical treatment and designing prosthetics for the patient, the lack of sub-surface information available in the models 1a, 1b pertaining to the patient's anatomy beneath the visible surfaces of the patient's oral cavity can hinder accurate planning and design.

To facilitate an accurate 3D socket model generation, in an embodiment each of the respective volumetric density scan data and the surface scan data from which the respective 3D surface models 1a and 1b are generated is submitted to a segmentation application. The segmentation application may be a remote service 244, or may be a local application (stored in local memory 204 and executed by one or more processor(s) 201. A segmentation processor processes each of the received surface scan data and volumetric density scan data to automatically recognize (via an image recognition function), extract and categorize individual recognized objects into labeled categories or classes (via a segmentation function).

For example, in an embodiment where the object is a tooth in a patient's oral cavity, the segmentation processor receives each of the intraoral surface scan data and the CBCT or other volumetric density scan data, and processes each of the scan data sets to recognize and label recognized objects as the individually identified teeth, gum, bone, and potentially other objects such as fillings, implants, etc. that are recognized in the received scan data. The segmentation processor tags recognized objects with corresponding object type labels associated with the object type (or classification) of the recognized object.

For example, the segmentation processor may recognize an object in the 3D model or scan data that corresponds to a tooth type 16 and assign to the recognized object an object type label "16" (or any unique label that classifies the recognized object as being of the unique object type corresponding to that patient's actual tooth 16). The segmentation processor recognizes and classifies (i.e., "labels") recognized data segments in the scan data into a plurality of individual segments and corresponding to various recognized object types.

Preferably, the individual segments comprise a 3D surface model representing the actual scanned object (e.g., a scanned tooth, portion of gums, bone, implant, etc.). In an embodiment, each segment corresponds to an individual object in the patient's oral cavity, and is labeled as such with an associated label. Each segment comprises a 3D model standing on its own, represented as a 3-di-mensonal triangular (or other polygonal) mesh.

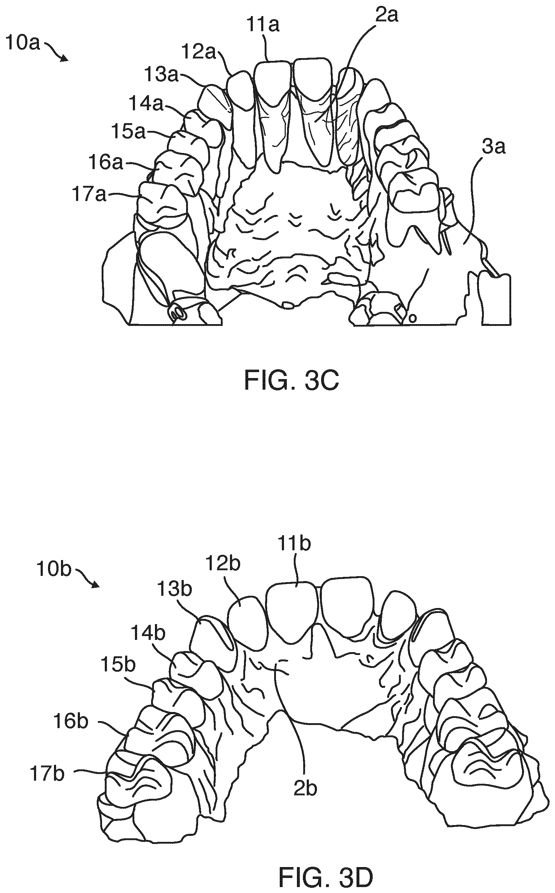

Referring to FIG. 3C, a segmentation processor may recognize representations of individual teeth 11, 12, 13, 14, 15, 16, and 17 in the volumetric density scan of the patient, and may segment each recognized representation of any of tooth 11, 12, 13, 14, 15, 16, and 17, gums 2, and bone 3, into corresponding independent segments 11a, 12a, 13a, 14a, 15a, 16a, 17a, 2a, and 3a, collectively forming a segmented volumetric density scan surface model 10a. Each segment is converted to an independent surface mesh, for example a 3D triangle mesh, and each segment may be independently selected (for example when presenting the segmented model in a graphical user interface (GUI) as discussed hereinafter.

Notably, because the segments 11a-17a, 2a, 3a were extracted from the volumetric scan data, each segment includes the full available information from the volumetric density scan. This means that objects (such as nerve canals) and portions of objects (such as the tooth roots) that cannot be imaged in a surface scan due to lying beneath the visible surfaces inside and outside of the patient's mouth, are still modeled in the volumetric density scan segments. Each volumetric density scan segment includes full object information (based on what is imaged in the volumetric scan data), even below the surfaces of the scanned anatomical area of the patient. Thus, each of teeth 11a-17a include the root information, which is clearly visible in the segmented model.

Similarly, referring to FIG. 3D, a segmentation processor may recognize representations of individual teeth 11, 12, 13, 14, 15, 16, and 17 in the surface scan of the patient, and may segment each recognized representation of any of tooth 11, 12, 13, 14, 15, 16, and 17 and gums 2 into a corresponding independently selectable segment 11b, 12b, 13b, 14b, 15b, 16b, and 17b and 2b, collectively forming a segmented surface scan surface model 10b. Each segment is converted to an independent surface mesh, for example a 3D triangle mesh.

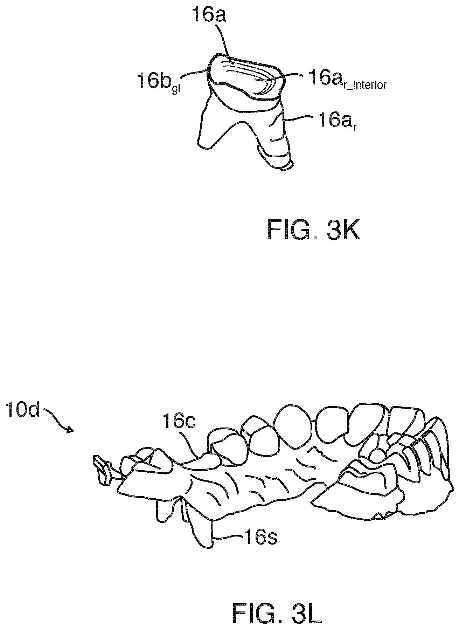

While the segmented model 10b includes all of the segments of the scanned anatomical area of the patient, each segment is an independently selectable 3D model of the corresponding scanned object. Accordingly, each segment 11b-17b, and 2b may be viewed alone, for example as shown in FIG. 3l (which shows tooth segment 16b alone corresponding to the patient's tooth 16). Tooth segment 16b includes only the portion of the tooth 16 that is present in the surface scan. Thus, tooth segment 16b represents only the crown of tooth 16 since only the crown (the portion of the tooth 16 that is above the gumline) is visible during the surface scan.

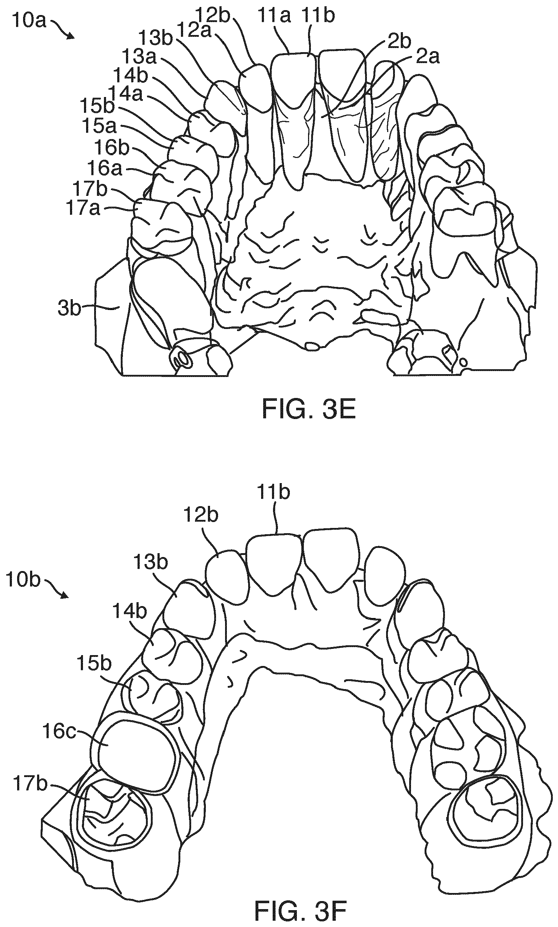

FIG. 3E shows the segmented volumetric density scan surface model 10a and segmented surface scan surface model 10b cross-mounted in a common 3-dimensional coordinate system. Independent imaging systems are typically used to capture each of the surface scan data and volumetric density scan data. For example, an intraoral scanner (IOS) may be used to capture the surface scan of the patient's oral area of interest, while a CBCT scanner may be used to capture the patient's volumetric density scan. Both scans are valuable in providing important information, and together supplement one another to provide a more complete image of the patient's actual oral situation. Surface scanners (such as optical scanners) can capture very high-resolution details of the visual topography of the patient's dentition but can only capture surface details and not internal details.

In contrast, volumetric density scans (such as CT or CBCT scans) can capture internal volumetric and density details of the patient's dentition, such as dimensions and density of the jaw, complete teeth (including roots), and nerve pathways. Together the surface scan and volumetric density scan can form the foundation of a dental treatment planning and prosthesis manufacturing process.

Because the independent imaging systems capture image data relative to the specific 3D coordinate system of the imaging system capturing the scan, to cross-mount the scans within a single view pane with having its own 3D coordinate system, both scans must be aligned with each other. This process is often referred to as scan matching or registration. Methods exist for aligning 3D meshes into a single 3D coordinate system.

In an embodiment, each of the surface scan data and the volumetric density scan data is segmented into 3D triangular mesh segments corresponding to individual teeth and jaws, followed by key point determination of corresponding tooth segments from each of the surface scan and volumetric density scan for each tooth, followed by alignment of the key points in a common 3D coordinate system. This process may be performed, for example, using CoDiagnostix^{™} dental implant planning software, offered by Dental Wings, Inc. (a Straumann Group company).

Notably in FIG. 3E, the positions of the respective segments (11a, 12a, 13a, 14a, 15a, 16a, 17a, 2a and 11b, 12b, 13b, 14b, 15b, 16b, and 17b and 2b) from respective segmented models 10a and 10b correspond to the same respective actual anatomical structures (11, 12, 13, 14, 15, 16, 17 and 2) in the patient's mouth. As can be seen, it is very important that the segments corresponding to portions of the same actual anatomical structure from each scan type (e.g., internal or surface) are mounted in the same 3-dimensional coordinate system. When they are properly cross-mounted, segments representing the same actual anatomical structure substantially coincide, as shown.

In an embodiment, a segmentation processor processes the scan data to identify and classify portions of the scan data into individual segments classified into anatomical structure types based on a set of labeled training data that includes multiple instances of each of the anatomical structure types. In an embodiment, the segmentation processor is a trained convolutional neural network (CNN) that has been trained on a large data set of scan images obtained from a large number of different people with different anatomical structure situations that include, or have missing, different teeth, gums, bone, and other natural and artificial (e.g., implants, prostheses, etc.) anatomical structures.

FIG. 3F illustrates the problem encountered when a tooth scan segment 16b (see FIG. 3D) is removed from the surface scan. As illustrated, removal of the tooth crown segment 16b results in a hole 16c in the model 10b where the crown 16b used to be. This is to be expected since the segmented surface scan 3D model 10b is generated based only on the surface scan data, which contains no bone or other beneath-the-surface information such as the root information of the teeth. Thus, when the tooth segment 16b is selected from the segmented surface scan model 10b (see FIG. 3D) and removed, there is no socket information available, and the surface scan model 10b has only a hole 16c where the tooth segment 16b was prior to removal.

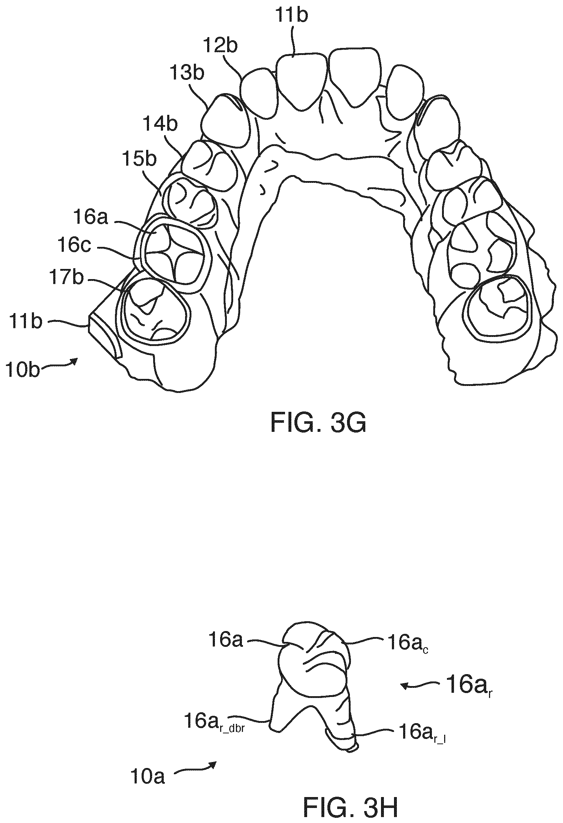

FIG. 3G shows the segmented surface scan 3D model after tooth segment 16b has been removed and the tooth segment 16a from the segmented volumetric density scan 3D model (from FIG.3C) is co-mounted in the same 3D coordinate system. FIG. 3H shows the tooth segment 16a, separate and apart from the other segments of the volumetric density scan model 10b. Tooth segment 16a includes a crown portion 16a_{c} and a root portion 16aᵣ. Notably, the root portion 16aᵣ of tooth 16 includes a trunk 16aₜ and three individual roots (a lingual root 16a_{r_l}, a mesio buccal root 16a_{r_mbs} (not visible in FIG. 3H), and a distal buccal root 16a_{r_dbr}. While the tooth 16 may have 3 individual roots, other teeth may have only one root, or may have two or additional roots. For simplification, the trunk and individual roots of any given tooth may herein collectively be referred to as "the root" of the tooth. Only the crown 16a_{c} is visible above the gumline in a surface scan. The root portion 16aᵣ is not visible above the gumline with the naked eye or to the cameras in an intraoral scanner.

FIG. 3l shows the individual tooth segment 16b (from the segmented surface scan 3D model 10b of FIG. 3G). As noted, the tooth segment 16b represents only the crown of the tooth 16 since only the crowns of the teeth are visible to the surface scan cameras (because they are above the gumline and can be seen with both the naked eye and also camera lense(s).

Notably, since both the surface scan and the volumetric density scan image the same area of interest, both scans include surface information pertaining to the same actual corresponding anatomical structures (assuming each scan scanned the same areas). This means that for the visible surfaces such as the crowns of the teeth, both the surface scan and the volumetric density scan will each include surface information or surface boundary information, respectively, pertaining to the crowns of the teeth.

Surface scans using optical sensors tend to produce much higher resolution images, resulting in surface 3D models with high detail. Volumetric density scans typically use modalities that either cannot be as accurate, or would be medically unsafe to make as accurate as an optical scan. For example, volumetric density scans generated using x-ray technologies including CT or CBCT modalities are based on x-ray radiation, and while highly accurate images could be obtained using a high dose of x-rays, to do so would be medically unsafe for the patient. Accordingly, CT and CBCT modalities used on patients are required to be set at very low levels of x-ray radiation to make them safer for humans. The tradeoff is that the accuracy of the images is lower. Accordingly, the crown surface data from the surface scan will generally include higher detail than the crown surface data from the volumetric density scan.

In order to generate a 3D model of the patient's oral situation after removal of a tooth, the application retains the root portion 16aᵣ of the volumetric density scan tooth segment 16a and removes the crown portion 16a_{c}. To do this, the application determines the gumline around the volumetric density scan tooth segment 16b based on the points along the lower edge of the surface scan tooth segment 16b.

FIG. 3J shows the surface scan tooth segment 16a and the volumetric density scan tooth segment 16b cross-mounted (both displayed within the same 3D coordinate system). As shown, the gumline 16b_{gl} is the set of points corresponding to the lower edge of the surface scan tooth segment 16b. Since the application knows the position of the gumline 16b_{gl}, it calculates the crown portion 16a_{c} as all points on the same side of the gumline (also called a cut-line) that the surface scan tooth segment 16b lies within the particular 3D coordinate system of the application.

Put simply, the application removes all points of the volumetric density scan tooth segment 16a that coincide or substantially fall within the same volume of the 3D coordinate system area as the surface scan tooth segment 16b (i.e., the crown) does, i.e. the corresponding portions of the volumetric density scan tooth segment and the surface scan tooth segment are co-represented. Put even more simply, the root portion 16aᵣ is obtained by subtracting 16b from 16a (and removing any outlier points, if necessary).

FIG.3K illustrates the root 16aᵣ upon removal of the crown portion 16a_{c} from the volumetric density scan tooth segment 16a. Since this is a surface model, only the exterior points of the segment are present in the 3D model; hence, when the crown portion 16a_{c} is removed from segment 16a, the inside 16a_{r_interior} is empty. The shape and form of the root portion 16aᵣ is defined only by points on the outer surface of the tooth root 16aᵣ as defined from the surface model of the individual segments obtained from the volumetric density scan.

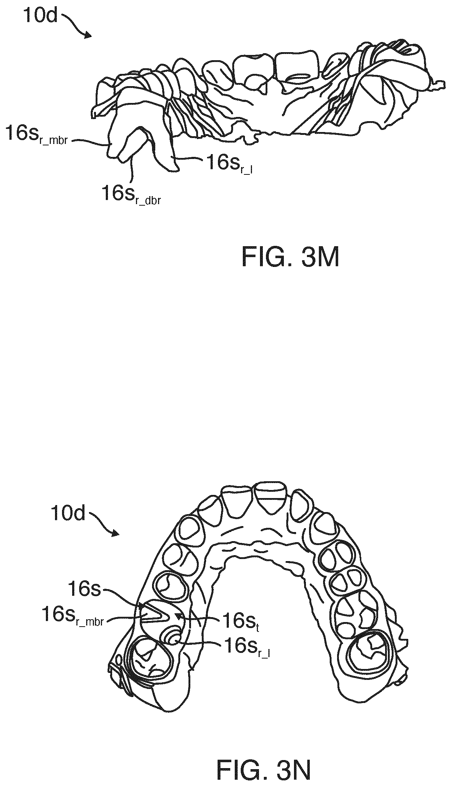

Accordingly, the contours of the inside of the root follow the contours of the exterior surface of the tooth root itself. The remaining root 16aᵣ may thus be displayed together with the surface model 10a (with the crown segment 16a removed) of FIG.3F to generate a surface 3D model 10d representing the patient's oral situation with tooth 16 extracted. This is shown in FIGS. 3L, 3M, and 3N. FIG. 3L presents the 3D model 10d substantially along the horizontal plane with a view of the lingual side to show the hole 16c and socket contour 16s. FIG. 3M shows the model 10d from another orientation, along the same horizontal plane as in FIG. 3l, viewed from the posterior side of the model. The socket contour is more visible from this point of view. FIG. 3N shows yet another view of the model 10d looking into the socket where the tooth 16 was virtually extracted. The contours of the socket 16s are visible, showing where two of the three individual root prongs were seated prior to virtual extraction of tooth 16.

The socket 16s includes the contours where the trunk, and each of the individual roots were seated prior to the virtual tooth extraction. As illustrated, the socket 16s follows the contours of extracted tooth's root, including a trunk socket portion 16aₜ and three individual root sockets including lingual root socket 16s_{r_l}, mesio buccal root socket 16s_{r_mbr}, and distal buccal root socket 16s_{r_dbr}, corresponding to lingual root 16a_{r_l}, mesio buccal root 16a_{r_mbr}, and distal buccal root 16a_{r_dbr}, respectively.

The volumetric density scan segment model of the tooth includes only the outer (boundary) surfaces of the tooth object, so it contains no information about the interior of the tooth itself. That is, for segmentation, the segmentation processor produces a 3D mesh of outer surfaces of the tooth without including any modeling of the inside of the tooth. For closed objects, such as a tooth, the 3D mesh model is also a closed triangular mesh (the number of edges and triangular shaped facets associated with any given vertex is equal). Thus, because the inside of the severed root segment 16aᵣ (i.e., segment 16 with the crown portion 16a_{c} removed) is empty, the inside surfaces of the root segment 16aᵣ follow the same contours as the outside surfaces of the root segment 16aᵣ. That is, the inside surfaces are merely the same exterior walls of the root segment 16aᵣ but viewed from the inside of the walls.

Removal of the crown portion 16a_{c} from the volumetric density scan segment 16a produces an open mesh (i.e., there exists at least one vertex in the mesh where the number edges associated with the vertex exceeds the number of triangular shaped facets associated that vertex). An edge facet as used herein is a facet whose number of adjacent facets (which share an edge) does not equal the number of edges of the facet. In the context of the removal of the crown portion 16a_{c} of the volumetric density scan segment 16a, the remaining portion of the volumetric density scan segment 16a, i.e., the severed root segment 16aᵣ, includes a set of edge facets along the margin line (where the crown meets the gumline), making it an open mesh. Since there is no information inside the severed root segment 16aᵣ, the inside surface of the open mesh is identical to exterior surface of the severed root segment 16aᵣ.

At any point after the segmented volumetric density scan surface model 10a and segmented surface scan surface model 10b have been cross-mounted in the common 3-dimensional coordinate system (cf. FIG. 3E), the user may inspect the oral cavity of the patient or indeed intervene in the oral cavity (such as by removing dental tissue with a dental drill). To do so, the user inserts the head of the appropriate dental tool into the oral cavity. Surface scanner 114 of system 100 scans the oral cavity and, as described above by reference to FIG. 2B, system 100 generates a display environment on GUI 120 of both the second dataset (i.e. at least a portion of the digital patient model) and a digital representation of surface scanner 114, with surface scanner 114 positioned and oriented according to its real-world position and orientation relative to the digital patient model.

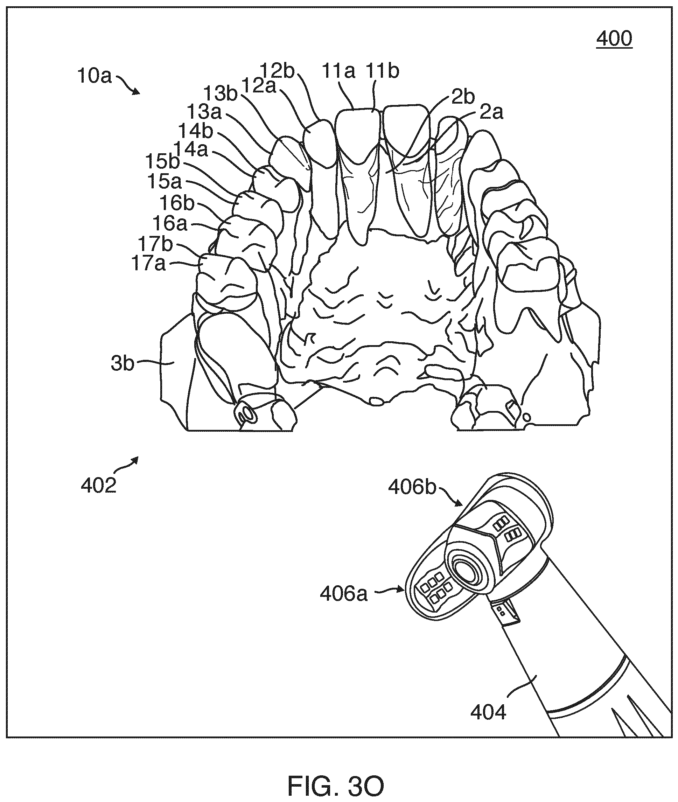

FIG. 3O is a schematical view of a display environment 400 of GUI 120, which includes a depiction of the digital patient model 402 and a digital representation 404 of surface scanner 114. In this example, surface scanner 114 is integrated into or includes a dental drill (shown without drill bit for clarity). Surface scanner 114 in this example comprises a pair of lateral extrusions to either side of the head of the dental drill, so these are also depicted (at 406a, 406b) in the digital representation 404 of surface scanner 114. (This arrangement maximizes the 3-dimensional sensitivity of the optical elements (i.e. light source(s) and light detector(s)) of surface scanner 114.)

As the user manipulates surface scanner 114 within the oral cavity, system 100 updates the view shown in display environment 400 of GUI 120 based on scan data continuously received by system 100 from surface scanner 114. As a consequence, the user can view precisely where surface scanner 114 is located within the oral cavity, including the position and orientation of the drill bit.

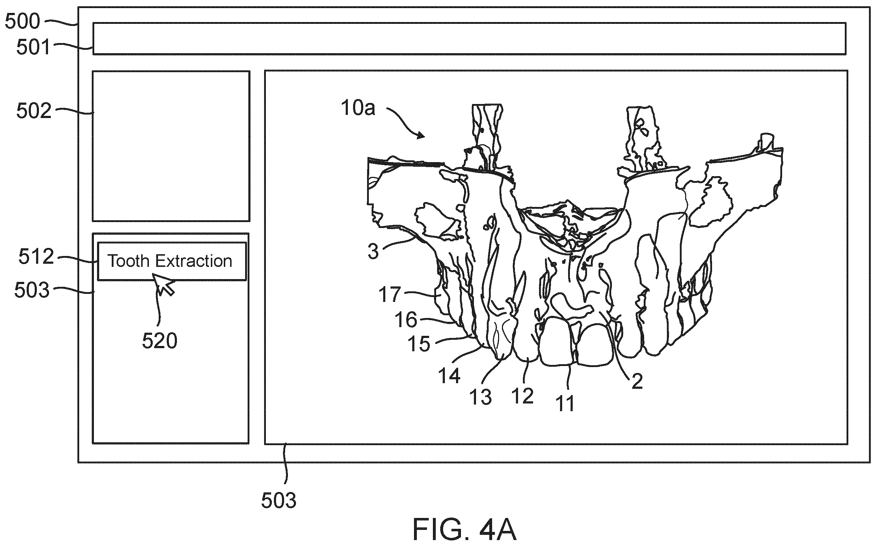

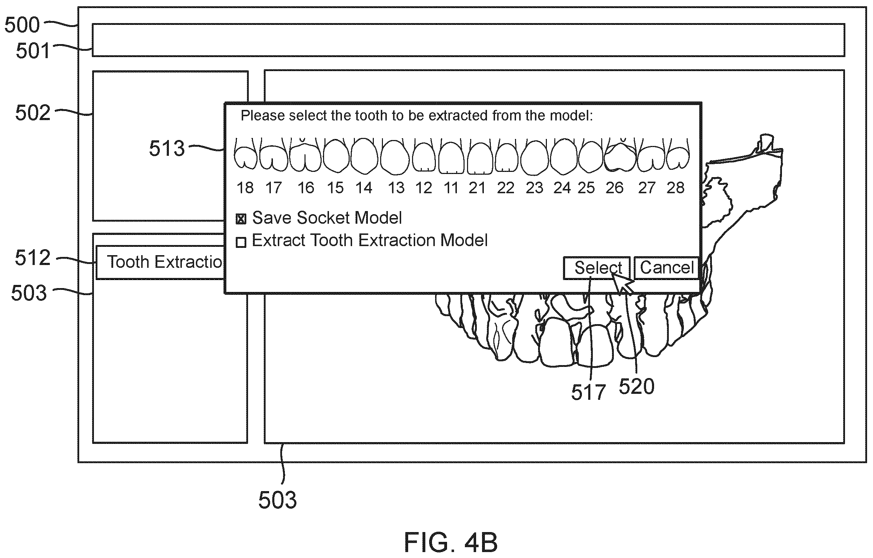

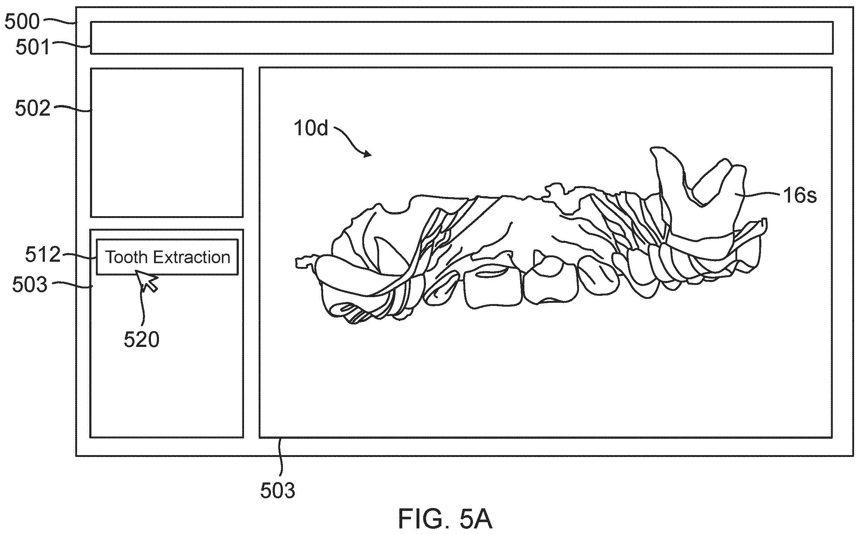

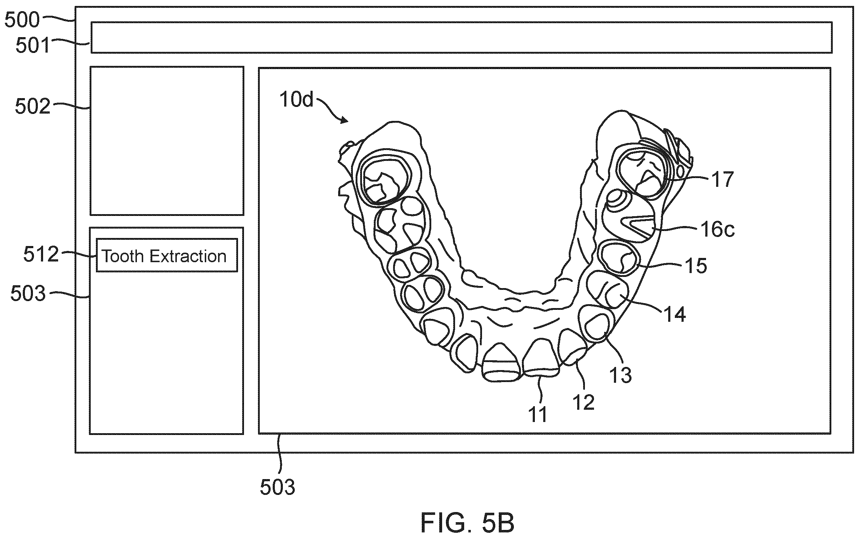

FIGS. 4A, 4B, 5A and 5B illustrate an exemplary embodiment of a display environment 500 of GUI 120 during various steps in a dental treatment planning workflow. The GUI display environment 500 may be displayed on an electronic display 118 of system 100 which implements a dental treatment planning application . The GUI display environment 500 includes control(s) 501 (not individually shown) to allow a user to select and load a patient's scan data into memory 106 of system 100 or on an external memory device (not shown) accessible through system 100 or which is accessible from a remote service 102 via network adapter(s) 122.

In the context of the described aspects of the invention, scan data includes surface scan data and volumetric density scan data of an anatomical area of interest of a patient. In an embodiment, both the surface scan and the volumetric density scan are obtained before the dental treatment planning; in other embodiments, one or both of the surface scan data and volumetric density scan data are obtained in conjunction with use of the dental treatment planning.

For example, remote surface scan service 130 may include an optical scanner application in communication with an optical scanner, and which communicates the optical scan data to system 100 during or after completion of an intraoral scan of the areas of interest in the patient's oral situation. Similarly, volumetric density scan service 132 may include a volumetric density scanner application in communication with a volumetric density scanner, and which communicates the volumetric density scan data to system 100 during or after completion of an intraoral scan of the areas of interest in the patient's oral situation.

As described above, system 100 manages the display of the graphical content in the GUI display environment 500 of GUI 120, including monitoring user input to the graphical controls received through user input device(s) 112, such as a mouse, keyboard, joystick, voice recognition, etc. User input may correspond to actions to be taken, such as invoking various application functions specific to the substantive features of the application or GUI functions for changing the layout or content of the features displayed on the display. More particularly, the front-end GUI displays user input controls and monitors user input associated with the functional controls. Upon receipt of user input associated with a user input control, the GUI invokes an appropriate function corresponding to the particular user input control and type and content of the user input. The GUI is also responsive to the backend process(es) which communicate with the back-end GUI, which in turn communicates with the front-end GUI to display, remove from display, and/or modify display of, information on the electronic display.

Such user selected functions may result in, without limitation, displaying, removing from display, and modifying display of: models, views, segments, and/or annotations, and displaying, removing from display, and updating the look of, various user controls and information displayed in the GUI display environment 500, and receiving and returning information to facilitate substantive functional features of system 100, including but not limited to: substantive treatment evaluation, substantive treatment planning, virtual performance of treatment or operations (such as tooth extraction, implant placement, prosthetic design and placement, etc.

Referring to FIG. 4A, the GUI display environment 500 includes global controls 501 such as file management, generic display controls, and other controls that are generic to the GUI display environment. For example, controls 501 may include file selection controls, file save/export controls, view pane format controls, etc. GUI display environment 500 also includes patient-data specific controls 502, such as arch and individual tooth model controls.

GUI display environment 500 includes functional controls 503, including a tooth extraction control 512. Tooth extraction control 512 is shown generically as a single control, but may comprises a plurality of controls such as guided dialog of pop-up display panels or other well-known GUI interactive techniques for displaying information and requests for information and for receiving user input. GUI 500 also includes and displays at least one view pane 503 for displaying therein a 3-dimensional model of a selected patient's 3D anatomical model of the patient's oral situation (or selected portions thereof), as obtained from the patient's scan data and as selected via selection controls in controls 502.

Controls 501 include one or more control(s) (not shown) which when selected, allows user selection of a patient's surface and volumetric density scan data from memory 106 of system 100. In an embodiment, when patient scan data is initially loaded, the GUI display environment 500 may display one or a plurality of view panes 503 (only one shown) in order to present on the display a visual overview of the patient's oral situation. In FIG. 4A, view pane 503 is shown displaying a 3D model of the volumetric density scan. Environment 500 may also include various additional views of the patient's oral situation based on the volumetric density scan data. For example, environment 500 may include a panoramic view pane, an axial view pane, cross-sectional view panes, and a tangential view pane (not shown).

An important objective of virtual tooth extraction tool, accessible via control 512, is to virtually represent the patient's oral situation upon virtual removal of one or more teeth or other objects selected for extraction. For example, if a tooth is virtually removed and displayed within the GUI display environment 500, the resulting displayed 3D model should include a representation of the tooth socket that become visible to the naked eye upon removal of the target extraction tooth. This ensures that system 100 generates a digital patient model, for display in display environment 500 with the digital representation of surface scanner 114, that is faithful to reality, but also modifiable by-for example-a virtual tooth extraction procedure to reflect a planned tooth extraction.

As shown in FIG. 4A, a user can activate a virtual tooth extraction tool by selecting a tooth extraction control 512 by moving, via a mouse (not shown), a graphical cursor 520 over the control 512 and mouse-clicking on the control 512. FIG. 4B shows an embodiment of a popup dialog 513 that is displayed in the GUI environment 500 when the Tooth Extraction control 512 is activated. As illustrated, the dialog may include a tooth selection map that allows a user of system 100 to select one or more individual teeth for virtual tooth extraction.

In an embodiment, the user may click on the individual teeth in the chart to select such teeth as a target extraction tooth. The user can optionally select, by selecting a respective selection click-box or radio button or other such selection feature, an instruction to save the socket model generated by the tool upon generation of the socket and/or an instruction to extract the tooth extraction model (which contains the model in the view pane 503 less the target extraction tooth/teeth plus the generated socket models for such target extraction tooth/teeth. When the user is done selection the target extraction tooth/teeth and save/extraction options, the user can click on the Select button 517 to invoke the tooth extraction tool.

FIG. 5A presents a posterior view (looking at the maxilla arch from the back towards the front of the patient) of the 3D tooth extraction model 10D, which more conveniently shows the 3D socket model 16s corresponding to the socket from which the tooth 16 is extracted. The socket model 16s is displayed together with the 3D surface scan model with the tooth 16 extracted. FIG. 5B shows the same model 10D from the inferior view (from the bottom looking up toward the maxilla). As illustrated, the tooth 16 is missing, but the interior 16c of the socket 16s is visible and it follows the contours of the root(s) of the extracted tooth 16.

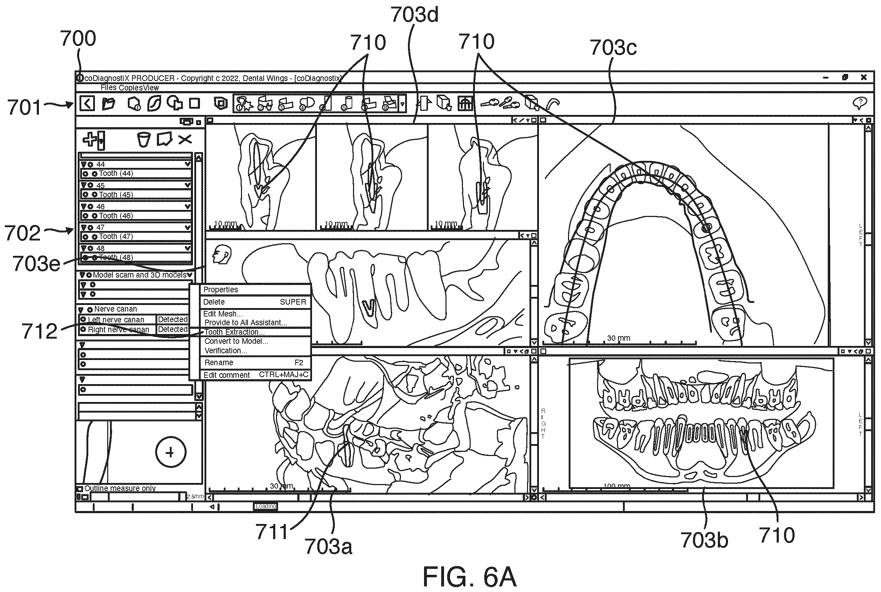

FIG. 6A depicts a GUI display environment 700 generated by system 100 (such as by display generator 250 of FIG. 1B) in which a patient's surface and volumetric density scans have been imported and loaded into memory 106 of system 100. FIG. 6A depicts the patient's dental situation after a dental professional has selected and virtually placed an implant. Techniques for virtually placing the implant in a virtual model of a patient's dentition are already known in the art, for example according to the use of CoDiagnostix^{®} Dental Implant Planning Software. In FIG. 6A, a virtual implant post 710 has been placed and is shown in various types of views in corresponding view panes of the graphical environment 700. In the example shown, implant post 710 is shown virtually placed in a cross-sectional view (view pane 703d, an axial view (view pane 703c), a panoramic view (view pane 703b), a tangential view (view pane 703e), and a 3D view (view pane 703a).

As shown in the various views in panes 703a through 703e, the placement of the implant is represented by the placement of an implant post or screw 710, which is the base portion of the full implant. A full implant includes an implant post 710, an abutment (not shown) that attaches to the implant post 710, and an abutment for a prosthesis or tooth restoration (also not shown), which may be a crown, bridge, or denture.

At the initial planning stage, only the implant post 710 need be virtually placed. The implant planning software application provides virtual implant placement guide(s) 711, which do not correspond to a physical component - they are visual indicators only that assist the dental professional in placing the implant at the correct angle. In FIG. 6A, the virtual guides 711 appear in the 3D view pane 703a as a long cylindrical rod with a central axis coincident with the central axis of the implant post 710 and having a diameter corresponding to the diameter of the abutment attachment socket inside the implant post. Preferably, the cylinder of the virtual implant placement guide 711 extends along the central axis above the occlusal plane of the teeth, such that the cylinder length is much longer than the cylinder diameter. Preferably, the guide 711 is displayed in a contrasting color to the colors used in the 3D model and other view panes so that the application user can immediately see the guide relative to the content in each view pane.

GUI display environment 700 includes a tooth extraction control 712, accessed in the exemplary embodiment by selecting a control from the view pane display controls 702 which corresponds to the portion of the patient's dentition in which the implant under consideration is placed. In the embodiment shown, the dental professional selects the lower arch control, right clicks on it to pull up a context menu and selects a tooth extraction control 712 from the context menu. The tooth extraction control may be selected to instruct the dental treatment planning application to automatically perform a virtual tooth extraction (using the principles described hereinabove). There exist many ways to implement the control that invokes the automated virtual tooth extraction tool - the key is to provide one or more controls that allow the user to invoke the tooth extraction workflow.

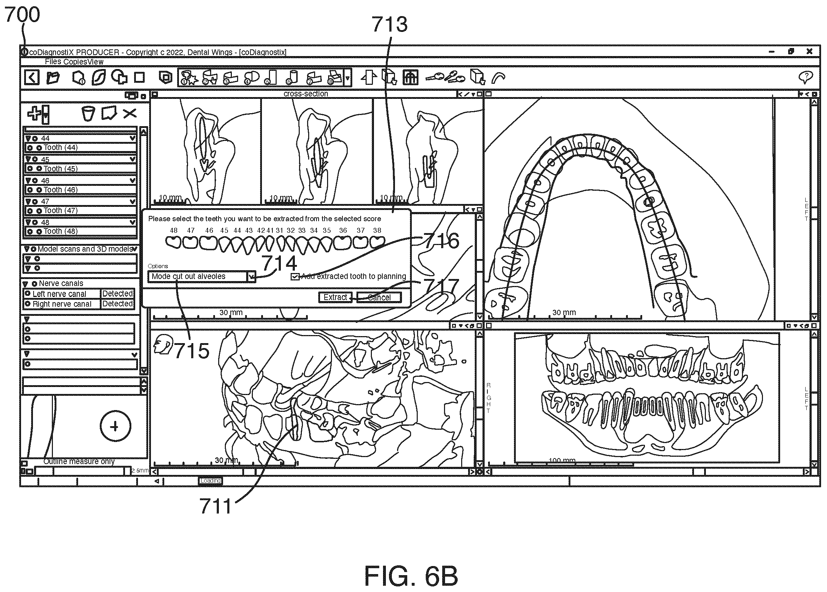

FIG. 6B illustrates a popup window 713 that appears in the GUI display environment 700 when the user selects the Tooth Extraction control 712. The popup window presents several options and user input controls for obtaining the inputs required by the virtual tooth extraction tool, including a tooth selection control 713, a mode control 714. In the embodiment shown in FIG. 6B, the tooth selection control 713 displays a set of selectable teeth icons corresponding to teeth in the selection portion of the patient's dentition as selected by the user in FIG. 6A. The user (i.e., the dental professional), may select the tooth corresponding to the tooth with the virtual implant post 710 is placed. In the example, the user selects tooth 35 corresponding to the tooth on the lower arch left side where the virtual implant post 710 is placed. For the mode, the user selects the "Mode: Cut out alveolus" 715 from the Options drop down menu 714, checks the check box control 716 to indicate that the extracted tooth should be saved as a separate file for future planning, and invokes the virtual tooth extraction tool by clicking on the Extract control 717.

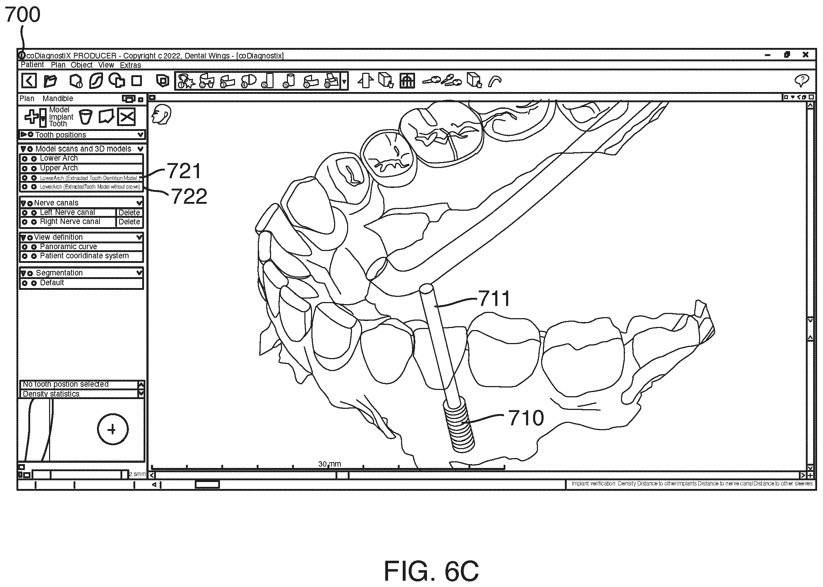

FIG. 6C shows a 3D surface model of the patient's dentition in view pane 703a. Upon completion, the virtual tooth extraction tool adds two 3D model files to the list of available model scans and 3D models in the controls section 702 of the GUI display environment 700. The user can select these files to display them in the pane. One file is an extracted tooth model (indicated at 721 in the file list). The extracted tooth model is created by the virtual tooth extraction tool according to the techniques described in connection with FIGS. 1 and 4, the extracted tooth model is a 3D surface model of the patient's dentition (including the previously placed implant post 310) with the selected tooth 35 removed from the model and a socket generated and included in the model in its place. The second file is an extracted tooth model (indicated at 722) in the files list, and is a model of the tooth 35, where the crown has been removed from the root.

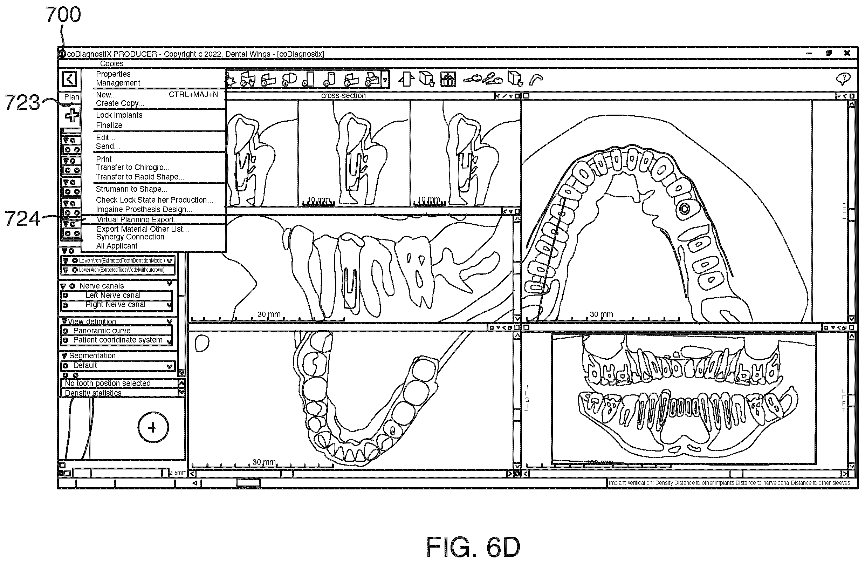

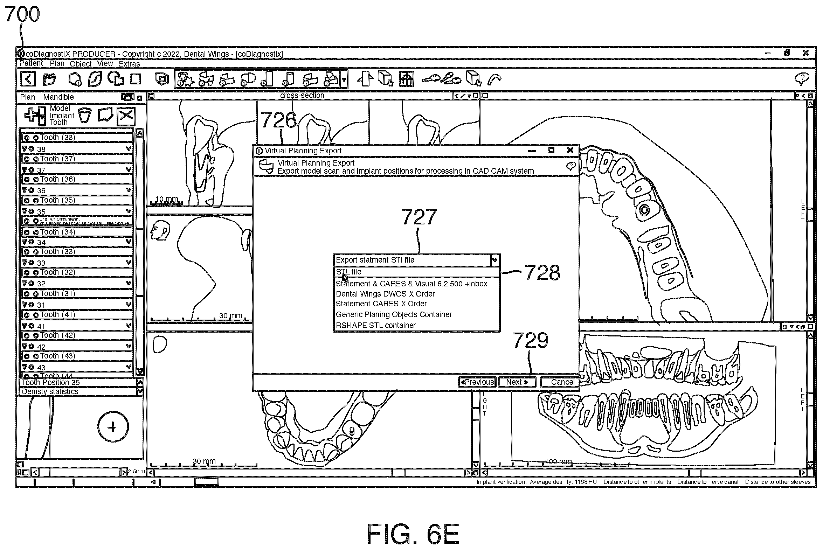

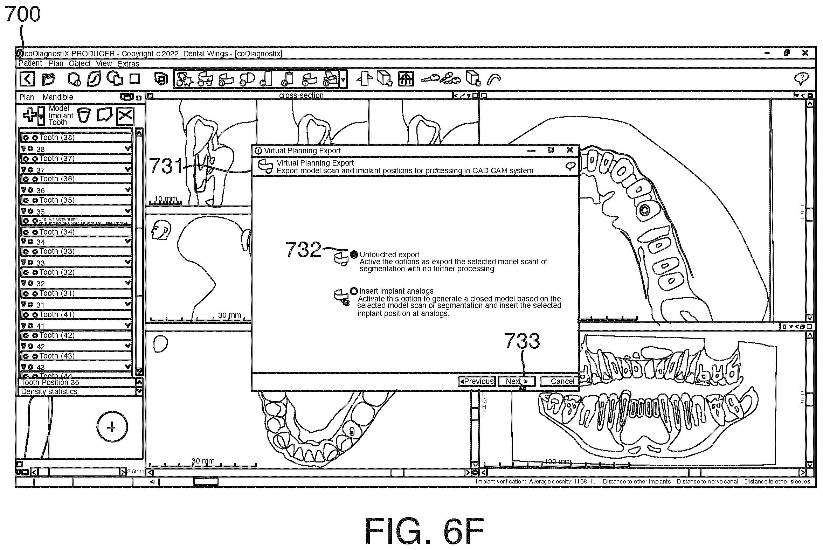

Once these files have been created, the user may click on the Plan menu 723 in the GUI display environment 700, as shown in FIG. 6D, and select a Virtual Planning Export control 724. In the next step, shown in FIG. 6E, the user can then select the format of the export file in a format selection control 727 of a popup window 726. In the example, the user selects an STL format option 728 and clicks on Next 729 to move to the next menu. In the next step shown in FIG. 6F, the user selects a button control 732 to activate an option to export the selected model scans or segmentations with no further processing.

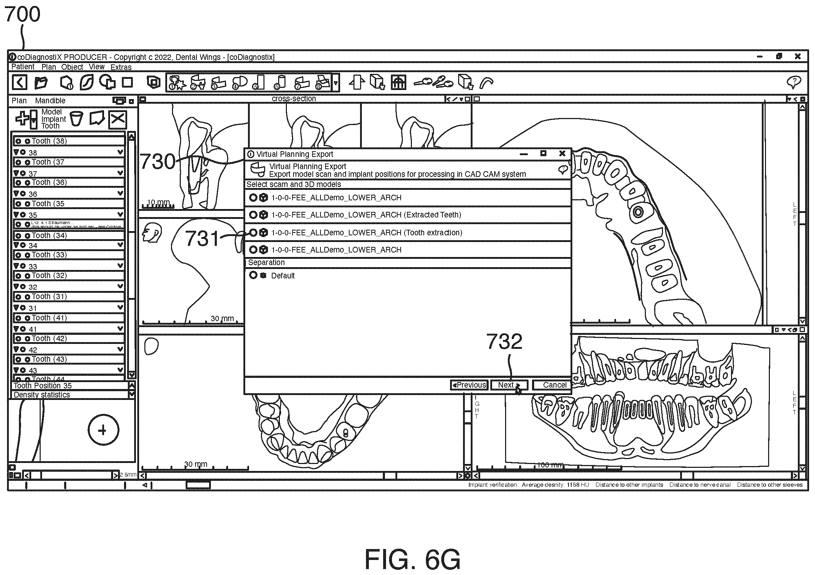

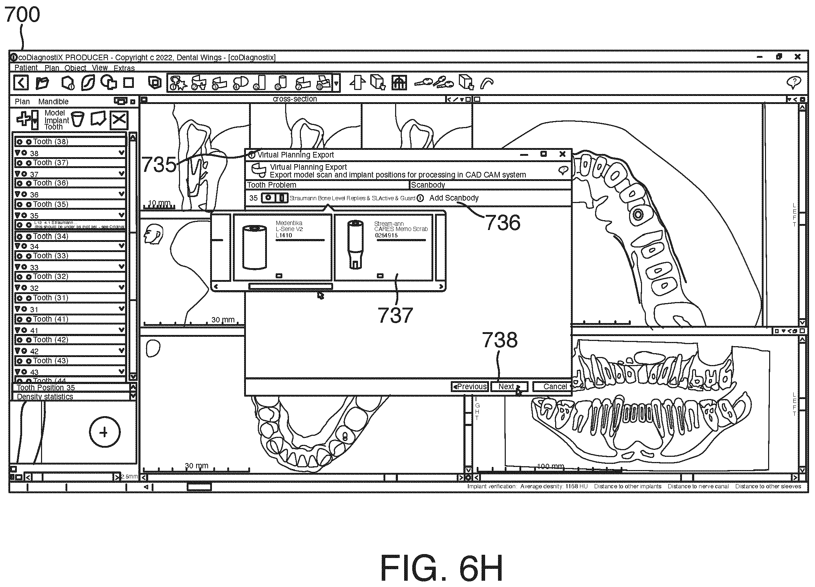

Clicking on the Next button 733, in FIG. 6G the tooth extraction file 731 is selected from the Export File selection popup window 730. Clicking on the Next button 732 brings up the implant selection popup window 735, shown in FIG. 6H, which offers a scan body selection control 736. The scan body selection control 736 includes a scan selection control 737 which displays and allows selection of a suitable scan body from a set of possible scan body types. The user can select a scan body from the menu of scan bodies to add the selected scan body to the model for export. The scan body will allow verification of the post-operative correct positioning of the implant (or other anchoring element), relative to the dental surfaces of the patient's dentition, after its implanting and, as the case may be, a healing period. This may be achieved by bringing the three-dimensional measurement data of a surface scan obtained from the person's post-operative oral cavity in registration with the 3-dimensional digital patient model, including the selected scan body.

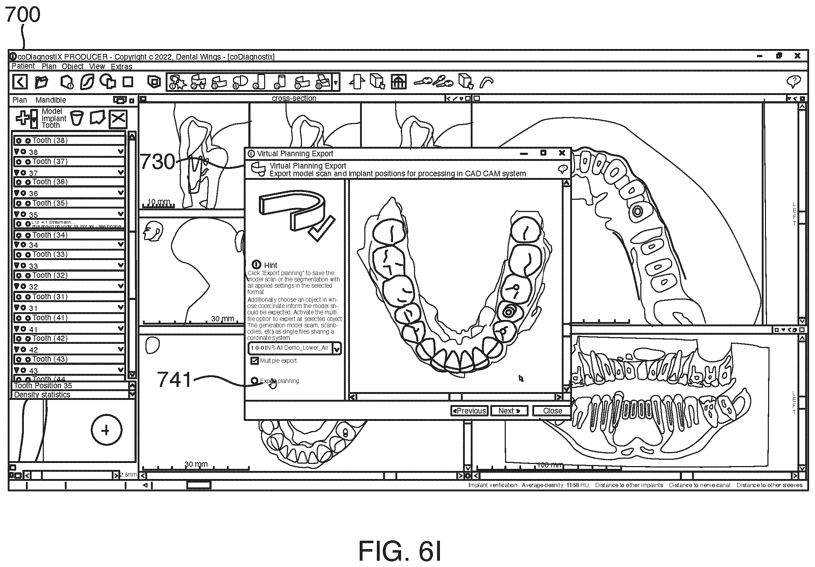

The user then clicks the Next button 738. In FIG. 6I, the user can select additional options, such as selection of the 3D coordinate system the model should be exported to, and selection of whether the exported objects should be exported as individual files sharing a coordinate system. Clicking on the Export Planning line 741 invokes the export function based on the selected options and parameters as selected by the user in the previous screens. The export files are saved in a known location in computer readable memory.

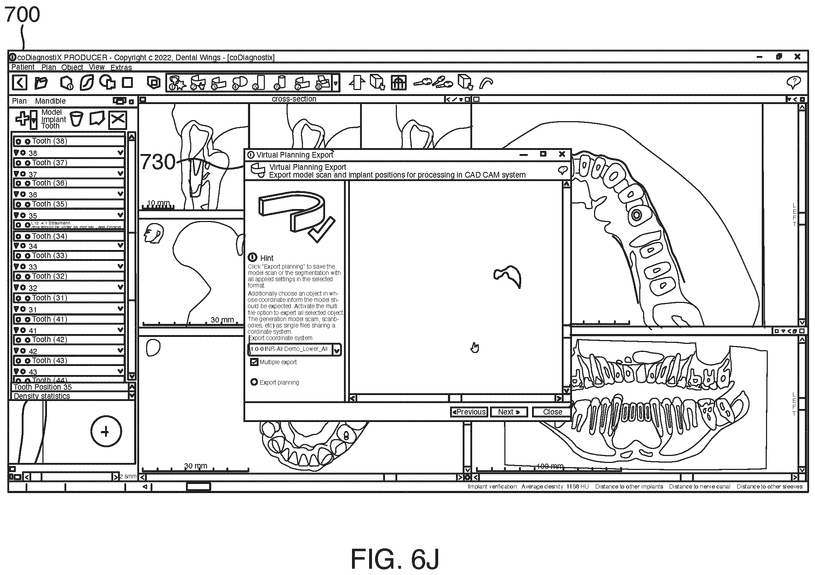

In FIG. 6J, the same process may be followed to export the surface scan crown segment of the virtually extracted tooth (and optionally the antagonist tooth crown segment - that is, the crown segment from the tooth in the opposite jaw which meets the targeted extracted tooth in the occlusal plane when the jaws are closed). It is important to ensure that the surface scan teeth segments are exported in the same coordinate system as the exported virtual tooth extraction model.

The resulting exported files comprise a 3D model of the patient's dentition with a virtual tooth extraction of the tooth where the intended implant replacement is to be placed. In place of the extracted tooth is a virtual socket where the virtual tooth was once seated. Included in the model is the virtual implant post placed in the socket where the dental professional placed it during the implant planning process.

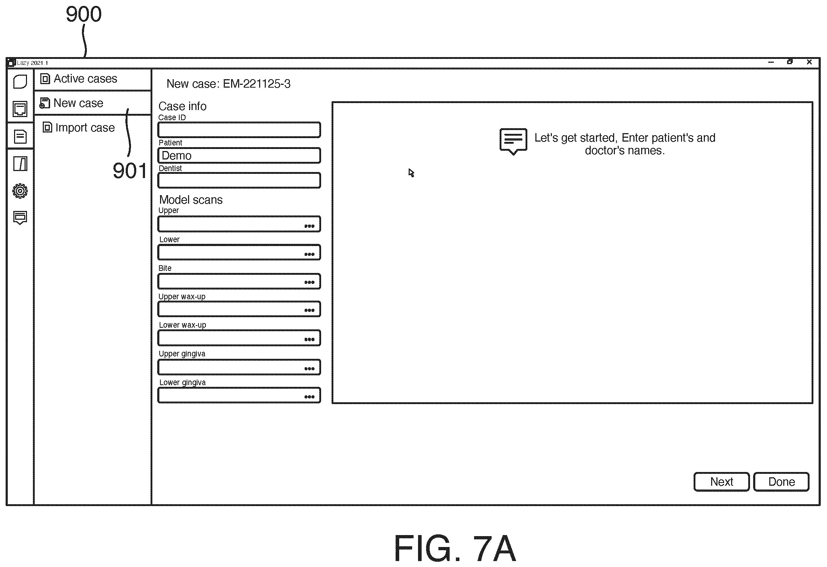

FIGS. 7A to 7T depict a GUI display environment 900 for a prosthetic design application, such as a computer-aided design (CAD) or computer-aided manufacturing CAM tool. In an embodiment, the prosthetic design tool is an application that operates in system 100 previously described by reference to FIGS. 1A & 1B.

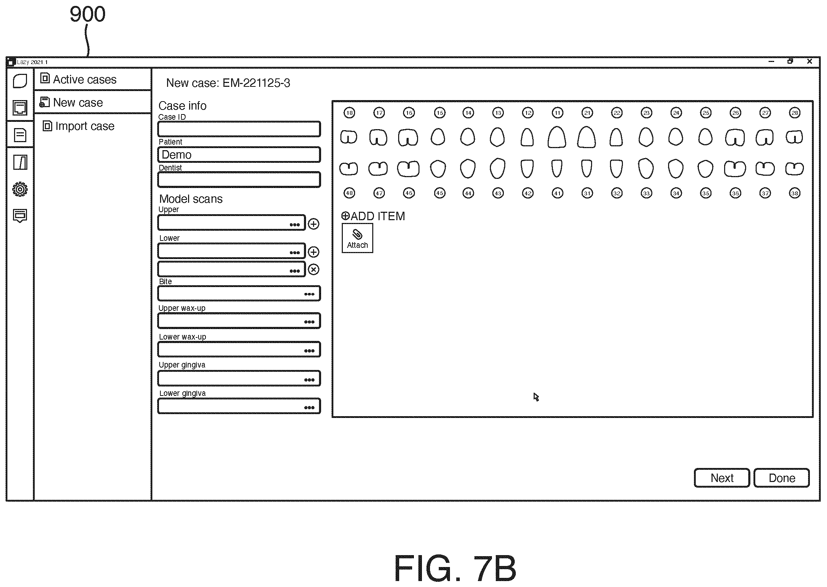

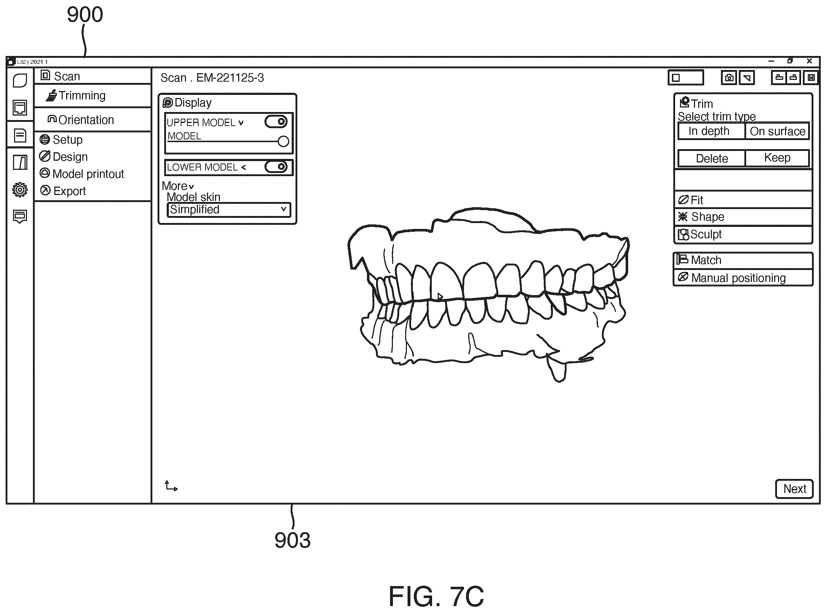

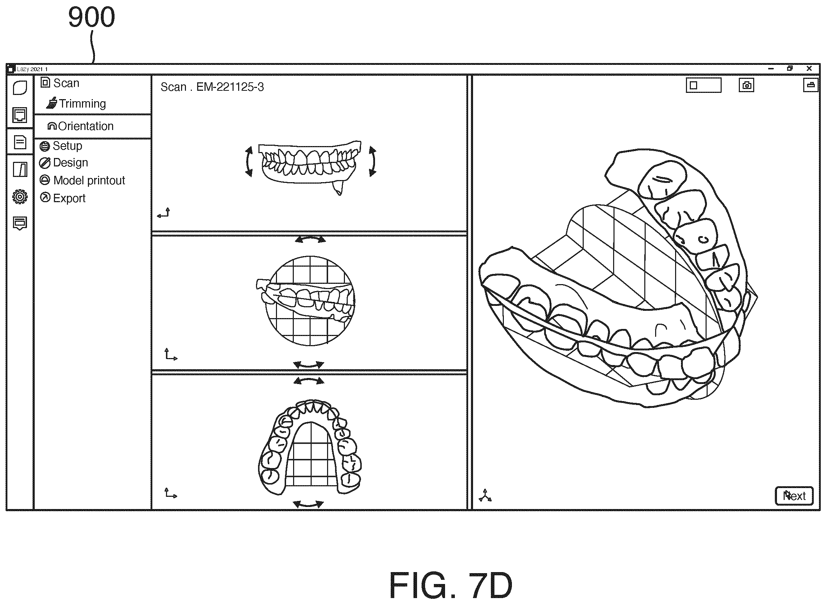

FIG. 7A is a GUI display environment 900 displayed on GIU 120 of electronic display 118 and having user input controls and display areas as discussed hereinafter. To design a prosthetic, a user begins a new case by clicking on control 901 (FIG. 7A), inputting case information (for example into text boxes Case ID, patient ID, and Dentist ID to be associated with the new case), and selecting and loading 3D models generated from patient scan data into memory for use by system 100. In the example, a tooth crown is to be designed. The user inputs the file names for the virtual tooth extraction files (in this case as the lower teeth model input) and the extracted tooth file (as the Lower wax-up) and loads the files into the system (for example by clicking on the Save button (FIG. 7B). System 100 displays the 3D model from the selected Virtual Tooth Extraction file(s) in a view pane 903 (FIG. 7C). If needed, system 100 provides controls in environment 900 for cleaning the scan (for example, to fill in holes where the scan data is incomplete, smooth scan lines, remove noise, etc.). Following, system 100 may provide tools to adjust the orientation of the model(s) to the occlusion plane if necessary (FIG. 7D).

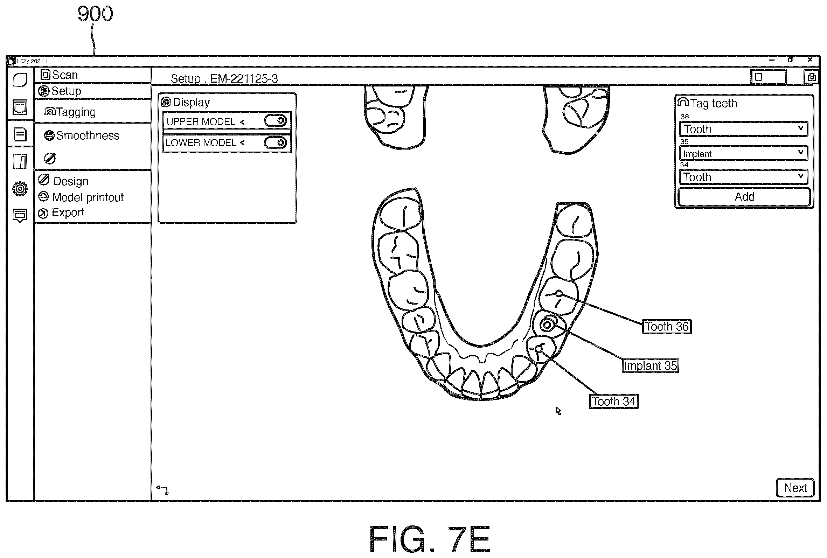

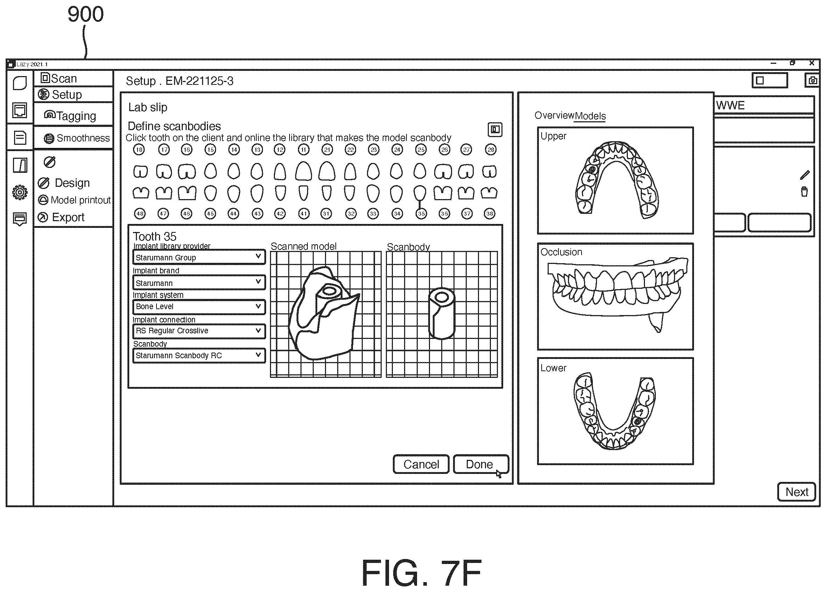

Prior to proceeding with the design, the user tags the teeth positions in the displayed model to indicate to system 100 the position(s) of the tooth or teeth for which a prosthetic is to be designed, and which teeth are adjacent to tooth/teeth for which the prosthetic is to be designed (see FIG. 7E). Next, in FIG. 7F, the user selects the platform (implant manufacturer, implant type and connection), and scan body. These selections should match the implant and scan bodies selected in the dental implant or treatment planning application and which the virtual tooth extraction files were generated based on.

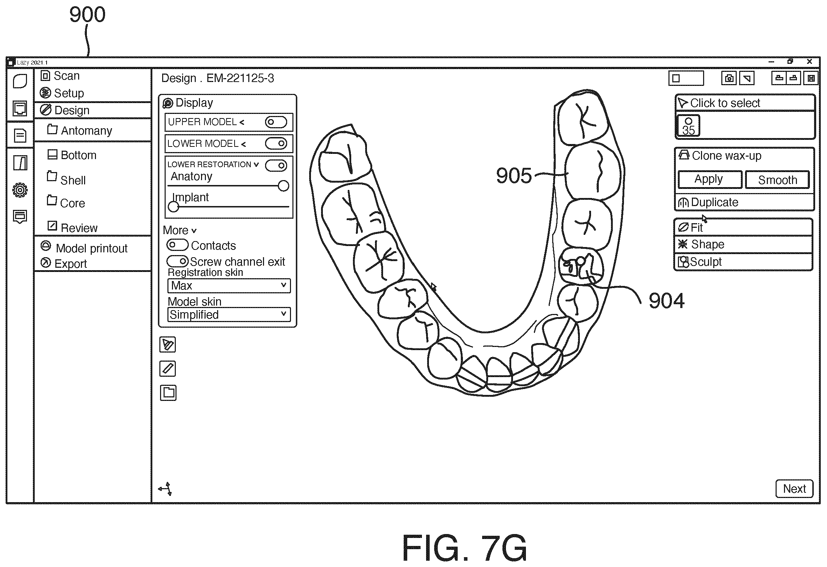

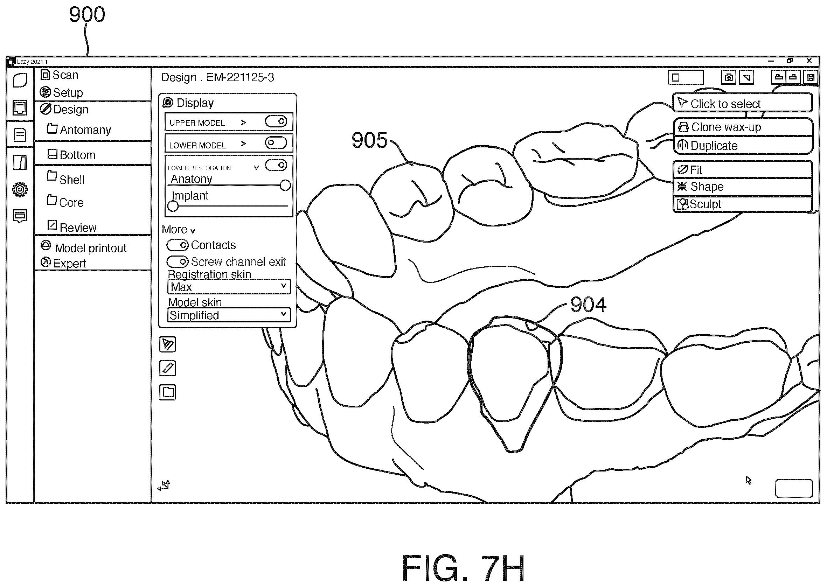

Once the setup is complete, the user can proceed to designing the prosthetic. In FIG. 6J, the surface scan crown segment from the 3D model was exported as an individual segment in a matching 3D coordinate system as the virtual tooth extraction model. Because the surface scan crown model was generated from an optical scan of the patient's original tooth (before actual extraction of the real tooth), the surface scan crown segment may be used as a digital wax-up model without having to again scan the patient's mouth. Since surface scan crown segment was exported as an individual segment matched to the same 3D coordinate system as the virtual tooth extraction model, the surface scan crown segment of the virtually extracted tooth can be mounted into the view pane with the virtual tooth extraction model and can be used directly by system 100 as the upper portion of the prosthetic crown. By importing the surface scan crown segment file as the wax-up file (FIG. 7B), the designer can select a Clone wax-up control (FIG. 7G) to instruct a prosthetic design application of system 100 to clone the wax-up model in the wax-up model file to serve as the prosthetic crown surface. Once the Clone Wax-Up tool clones the wax-up model as the prosthetic, the user can then perform fine adjustment tuning of the prosthetic shape, via fitting, shaping and sculpting controls available in the GUI display environment 900. The environment 900 also includes controls to rotate and change the view of the model displayed in the view pane 903 so that the user can view the prosthetic 904 from all angles. For example, in FIG. 7H, the model 905 is rotated so that the prosthetic 904 can be viewed from the buccal side. This allows the designer to make adjustments to the prosthetic by viewing the prosthetic in situ within the virtual tooth extraction model 905.

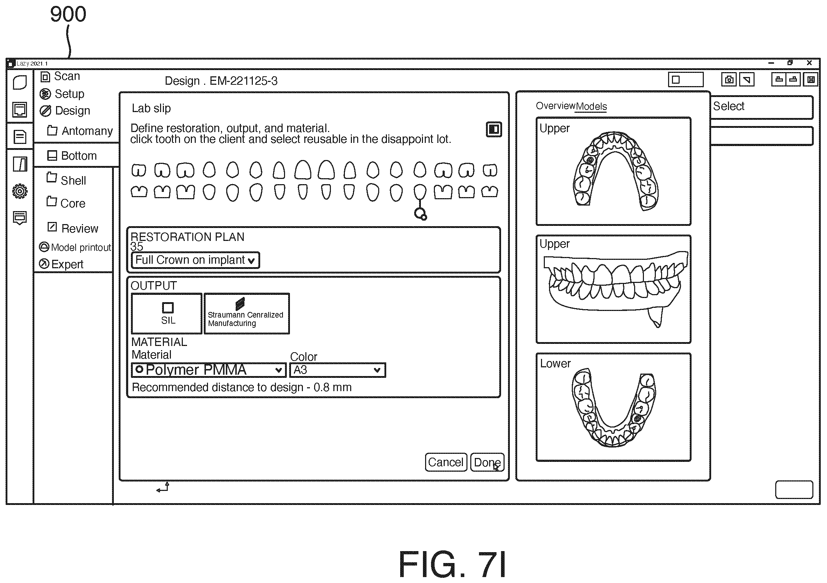

Once the visible surfaces of the crown are designed based on the extracted surface scan crown segment obtained during the tooth extraction process, the user may then design the bottom of the prosthetic. System 100 may provide controls for the designer to input restoration specifications (see FIG. 7I), such as material type, color, as well as whether what should be output (e.g., output an STL file, output an order placement (which can be a direct communication to a remote manufacturing facility).

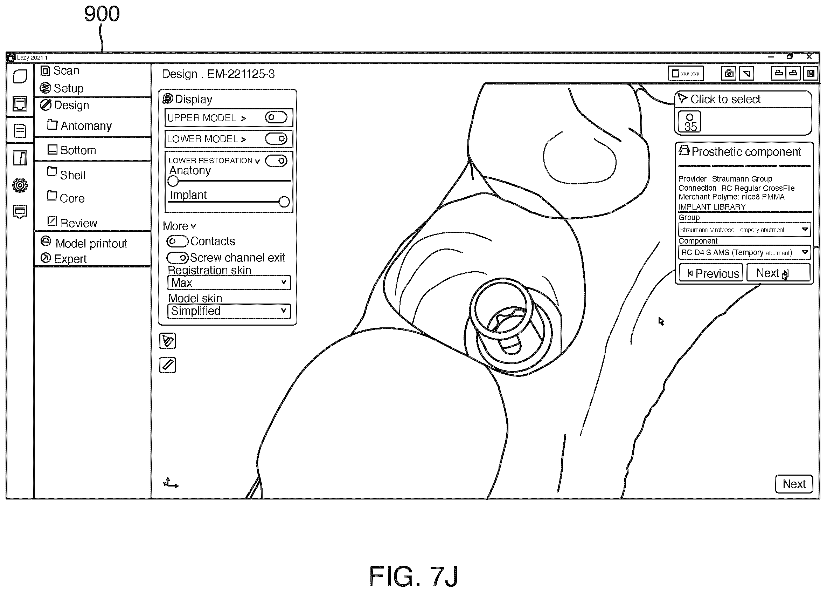

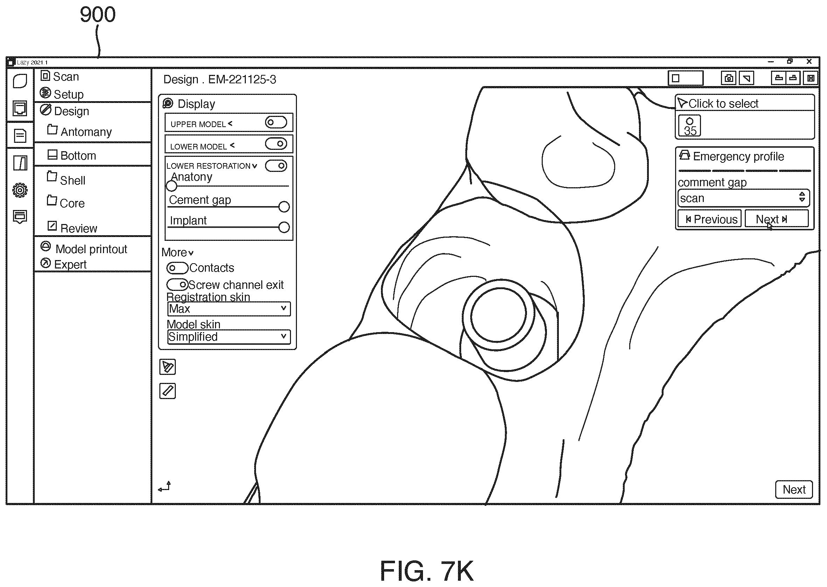

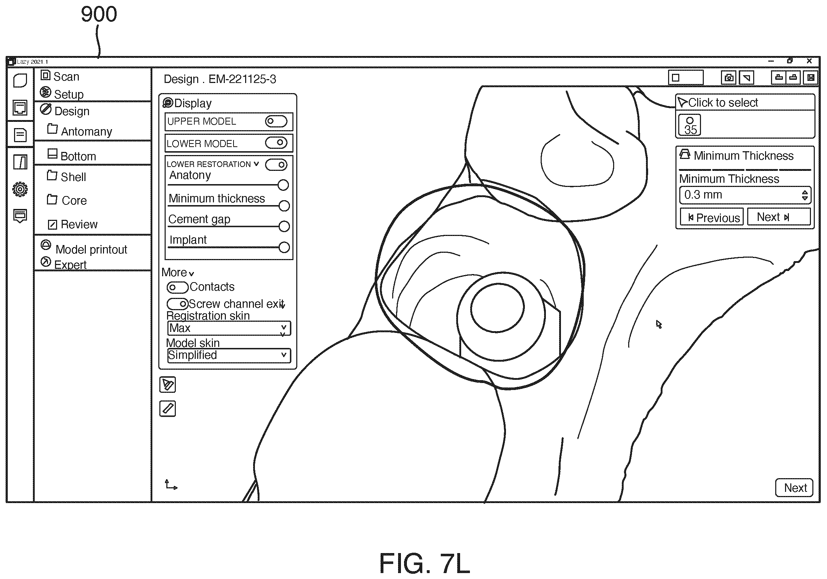

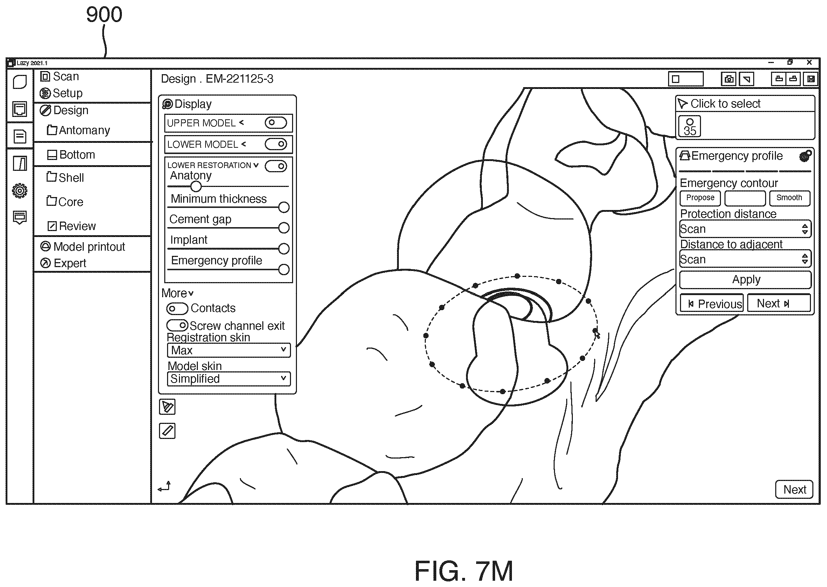

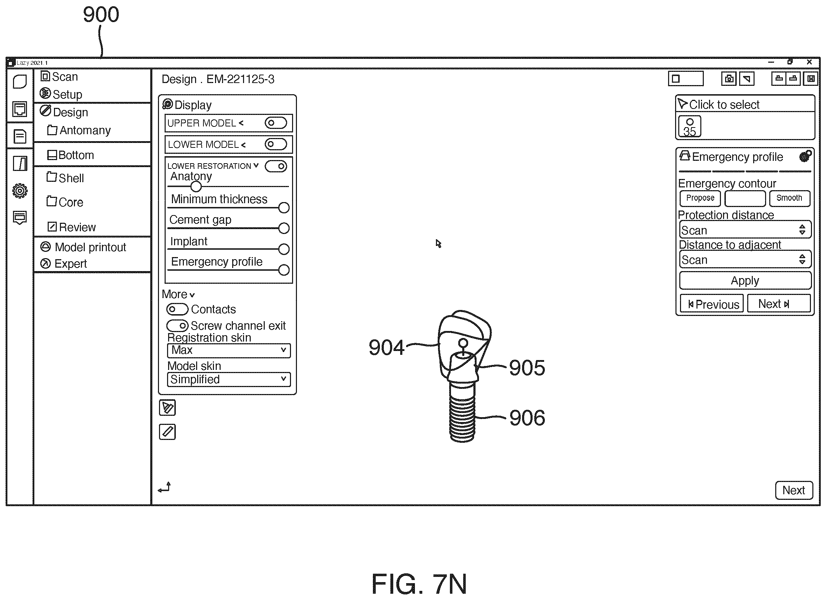

In FIG. 7J, the virtual tooth extraction model 905 is displayed in the view pane 903. Since the example case in FIGS. 7A to 7T is a new case for design of a prosthetic design for an implant, and the virtual tooth extraction model 905 was exported with the implant placed in the model (for example using the process described in connection with FIGS. 6A to 6J), the virtual tooth extraction model 905 includes a virtual implant post. In this example, a temporary abutment is selected and automatically virtually connected to the implant post and displayed as shown. The user can then select the thickness of the cement gap (FIG. 7K), set the thickness of the material (FIG. 7L), and display the prosthetic 904 within the model 905, which includes the socket contours (or "emergence profile") (FIG. 7M). The user can also then turn off display of the model 905 to display only the prosthetic (FIG. 7N).

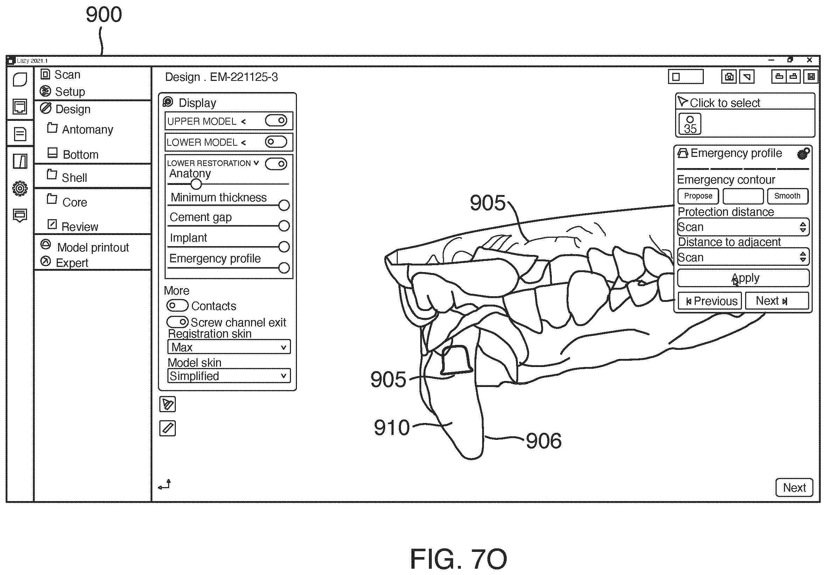

FIG. 7O shows the model 905 turned back on for display (see the on/off buttons for display of various models in FIG. 7O), and rotated to get a good side external view of the socket 910. With the anatomy transparency level set low (in order to view the implant post and abutment inside of the socket 910, it becomes clear that one can design an anatomically correct prosthetic 904 that conforms to the patient's oral anatomy by using the socket contours to guide the design of the lower crown portion of the prosthetic to fit against the contours of the inside of the socket 910. In this regard, the prosthetic design application provides controls to adjust the shape and fit of the lower portion of the crown.

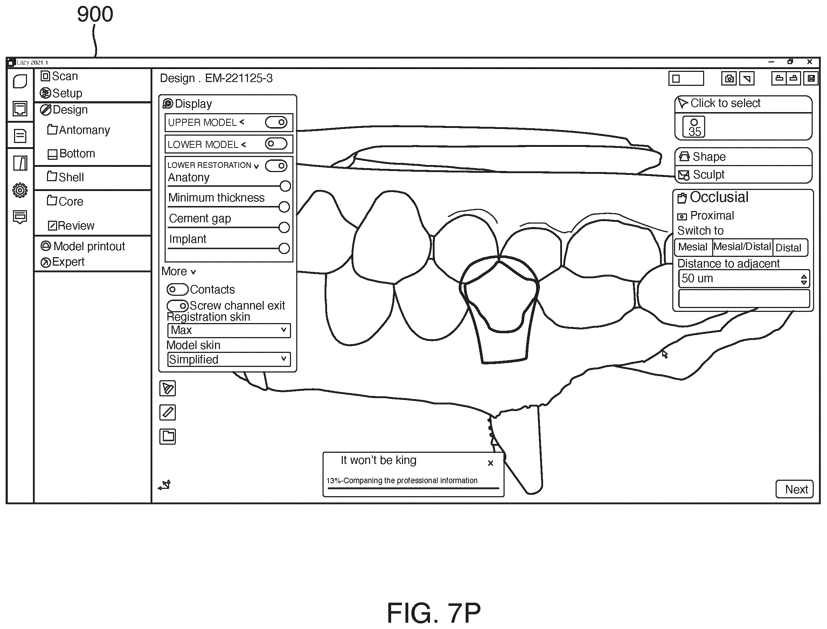

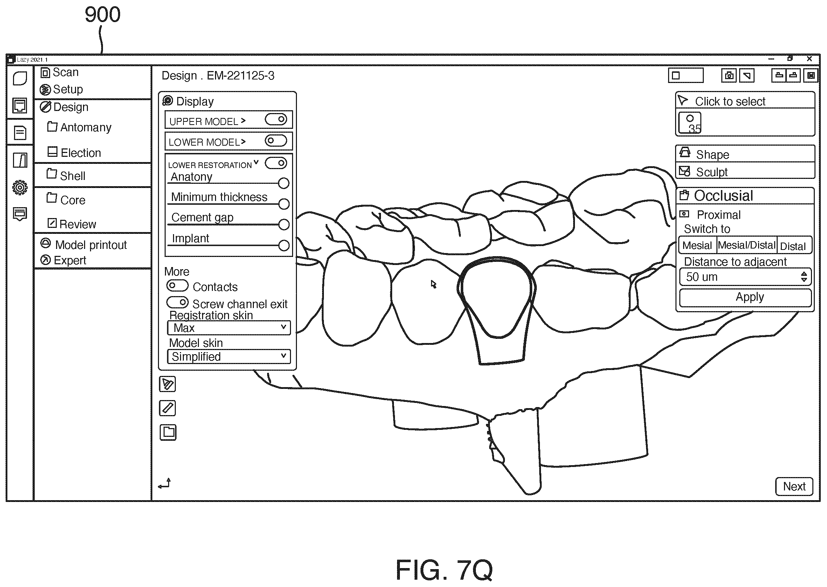

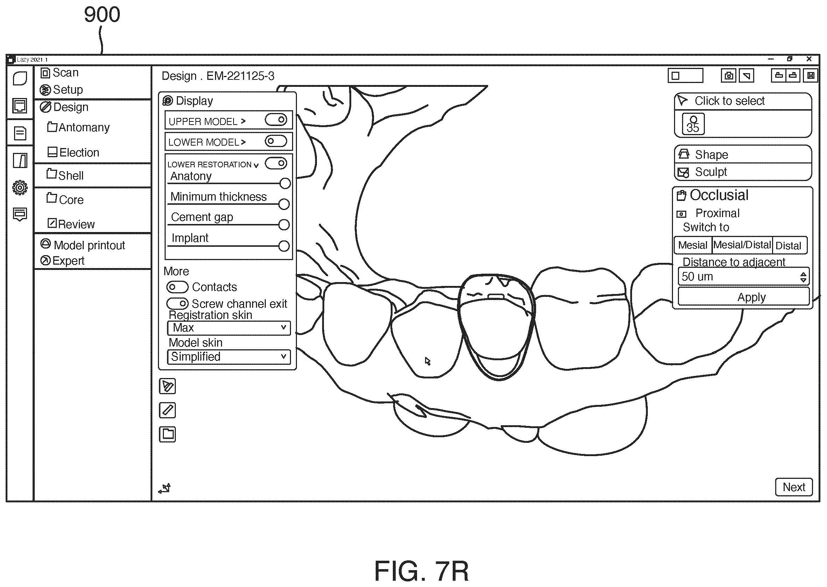

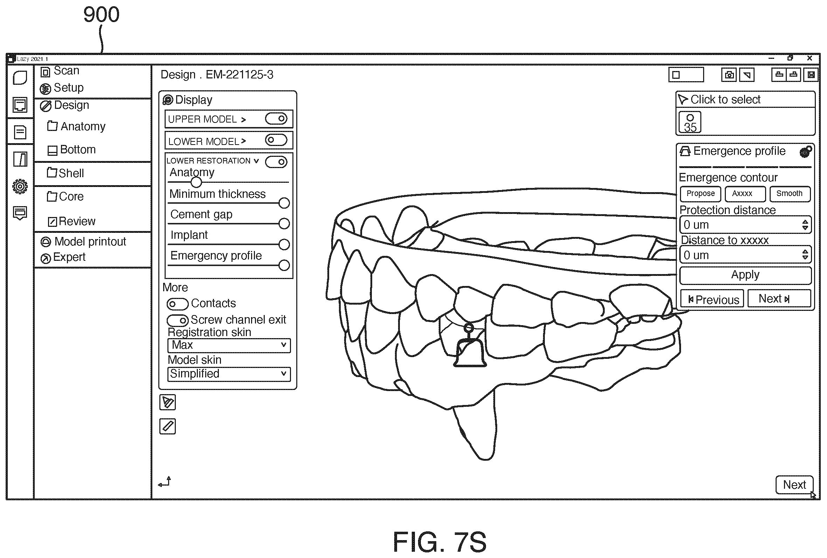

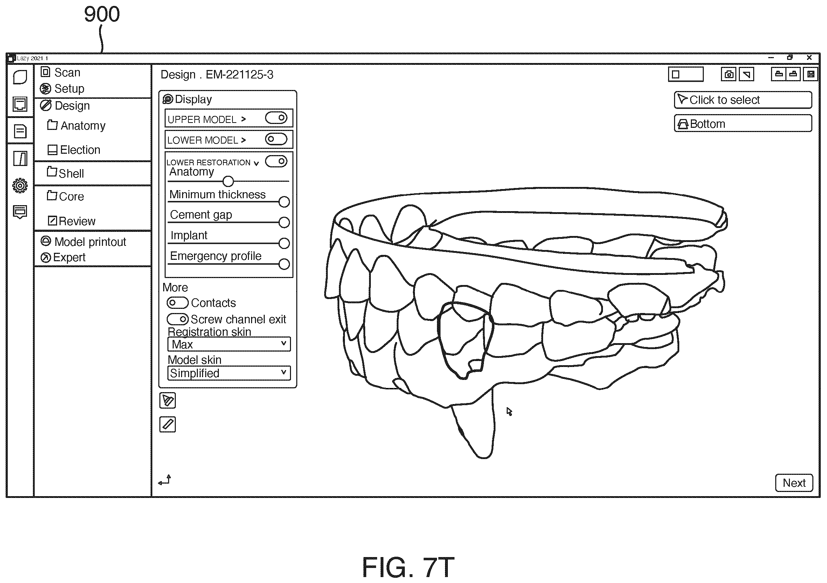

Once the bottom of the crown prosthetic is shaped to conform to the contours of the socket 910, the designer can move on to specifying the shell of the prosthetic. The application may automatically calculate the proximal distance information between the prosthetic surfaces and the teeth on either side of the prosthetic (when it is virtually attached to the virtual implant post 906) (FIG. 7P) and generate a shell surface (viewed from the buccal side in FIG. 7Q and looking down at the occlusal plane in FIG. 7R). FIG. 7S shows the final prosthetic design within the model with both the upper and lower jaws with the anatomy model partially set to transparent in order to partially see the implant post 906, abutment, and prosthetic crown. The user can easily check the placement and shape of the prosthetic through visual inspection of the model. FIG. 7S. FIG. 7T shows the same model and orientation as in FIG. 7S, but with the anatomy at full opacity. The workflow will continue though the usual steps before sending the crown to production.

Upon completion of the prosthetic design, the design files may be exported and used to manufacture the design. In an embodiment, the exported design files may be sent to a manufacturing facility or a remote manufacturing service. In an embodiment, the exported prosthetic design files may be used to generate 3D printer instructions for submission to a 3D printer that is responsive to such instruction to 3D print the prosthetic.

The above-described aspects and embodiments of the invention provide multiple advantages in virtual socket visualization, anatomical treatment planning and anatomical prosthetic design. According to one advantage, anatomical treatment professionals and prosthetic designers can plan treatment and design anatomically accurate prosthetics based on an accurate 3-dimensional model of socket that is the site of treatment planning and prosthetic design and which models a patient's specific socket anatomy.

The model may be generated prior to extraction of the anatomical object from the patient which allows accurate treatment planning and design of the prosthetic prior to, or concurrently (i.e., in parallel) with, the actual surgical extraction of the anatomical object from the patient. This means that the patient may visit the treatment professional for as few as a single visit. During as few as one or two office visits, the patient's anatomical area of interest including the anatomical object targeted for extraction may be scanned. The scan data may be imported into a digital treatment planning tool that includes a virtual socket model generation tool and/or virtual object extraction tool, each of which generates a virtual socket model that is included in the display of a virtual anatomical model of the anatomical area of interest of the patient (i.e., the area that includes the target extraction object and adjacent anatomical objects or features).

With the virtual socket model included in the displayed virtual anatomical model, the treatment professional can more accurately virtually place an implant or other treatment body in the virtual socket model, design and print 3-D printable surgical guides, and export the virtual anatomical model the virtual socket model for use in a separate prosthetic design software tool to design the anatomically accurate prosthetic. If the treatment professional has access to immediate prosthetic manufacture equipment, the prosthetic can be manufactured while the patient is still in the office. Otherwise, the prosthetic can be sent to a lab for manufacture and the patient can return to the office when the anatomical area surrounding the implant has healed sufficiently to attach the prosthetic to the implant.

## Claims

1. A computer implemented method (100) for generating, by one or more computer processors, a 3-dimensional model (10D) for use in assisted navigation of the oral cavity of a patient, the method based on at least a first scan of an anatomical region of the patient's oral cavity, the method comprising:
receiving a first dataset including surface boundary information from the first scan and generating a 3-dimensional digital patient model from at least a portion of the first dataset;
performing a 3-dimensional surface scan of a part of the intraoral cavity using a 3-dimensional surface scanner and thereby acquiring a 3-dimensional surface scan, the 3-dimensional surface scan including a region of interest;
matching the acquired 3-dimensional surface scan to at least a portion of the 3-dimensional digital patient model;
determining, employing the acquired 3-dimensional surface scan and the digital patient model, a spatial transformation configured to align or bring into a predetermined degree of registration the acquired 3-dimensional surface scan and the 3-dimensional digital patient model;
determining a position and orientation information of the 3-dimensional surface scanner relative to the 3-dimensional digital patient model using at least the transformation; and
generating a second dataset including the position and orientation information of the 3-dimensional surface scanner relative to the 3-dimensional digital patient model.

2. A method according to claim 1, wherein the second dataset further includes at least a portion of the 3-dimensional digital patient model.

3. A method as claimed in claim 1 or claim 2, wherein the surface boundary information of the first dataset includes surface segments, the surface segments having labels associated therewith identifying the type of the tissue, each surface segment represents, the type of tissue being selected from a group including gum tissue, bone tissue, and dental tissue.

4. A method as claimed in claim 3, comprising identifying elements of the anatomical region of the patient's oral cavity in the 3-dimensional digital patient model comprising retaining labels associated with surface segments identifying the type of tissue in the process of generating the 3-dimensional digital patient model.

5. A method as claimed in any one of the preceding claims, wherein the second dataset further comprises a digital representation of the 3-dimensional surface scanner in a common reference frame, the digital representation of the 3-dimensional surface scanner being positioned and oriented according to the position and orientation information.

6. A method as claimed in any one of the preceding claims, comprising generating the second dataset as a 3-dimensional output model.

7. A method as claimed in claim 6, comprising the navigation system receiving a dental treatment or inspection plan, determining a region of interest in the 3-dimensional output model based on the dental treatment or inspection plan, and identifying the region of interest in the 3-dimensional output model.

8. A method as claimed in any one of the preceding claims, comprising transferring the second dataset to a visualization system configured to visualize at least a portion of the second dataset using a 3-dimensional or a pseudo-3-dimensional display device.

9. A method as claimed in claim 8, comprising the visualization system augmenting or overlaying the display of the portion of the second dataset with a view of the patient's oral cavity.

10. A method as claimed in any one of the preceding claims, comprising acquiring and updating the 3-dimensional surface scan continuously or in real time.

11. A method as claimed in any one of the preceding claims, comprising transferring the second dataset to a robotic navigation system configured to control navigation of a dental instrument, such as a dental drill, a dental probe, or another type of dental instrument, based on the position and orientation information included in the second dataset.

12. A method as claimed in any one of the preceding claims, wherein the 3-dimensional surface scanner comprises, is integral with or is mounted on a dental instrument, such as, a dental drill, a dental probe, or another type of dental instrument.

13. A method as claimed in any one of the preceding claims, wherein matching at least a portion of the 3-dimensional digital patient model to the acquired 3-dimensional surface scan employs only surface boundary information of the first dataset including segments having been labeled as dental tissue.

14. A method as claimed in any one of the preceding claims, wherein the surface boundary information from the first scan is determined from a surface scan and/or a volumetric density scan of the anatomical region of the patient's oral cavity.

15. A method as claimed in any one of the preceding claims, wherein the digital patient model does not include a digital representation of a marker element.

16. A system for generating a 3-dimensional model for use in assisted navigation of the oral cavity of a patient, the system (100) comprising:
at least one computer processor (104);
a storage unit (106);
a 3-dimensional surface scanner (114) for performing a 3-dimensional surface scan of a part of an intraoral cavity of the patient; and, optionally, a display device and/or a robotic navigation (assist) system,
wherein the storage unit (106) stores instructions which, when executed by the computer processor (104), implements a method according to any one of claims 1 to 15.

17. Computer program code which, when executed by a computer processor (104) in communicative connection with a 3-dimensional surface scanner (114) for performing a 3-dimensional surface scan of a part of an intraoral cavity of a patient executes the method of any one of claims 1 to 15.
